(19) Europäisches Patentamt
European Patent Office
Office européen des brevets

(11) Numéro de publication: **0 199 636**
**B1**

(12) # FASCICULE DE BREVET EUROPEEN

(45) Date de publication du fascicule du brevet: **08.02.89**

(21) Numéro de dépôt: **86400785.1**

(22) Date de dépôt: **11.04.86**

(51) Int. Cl.⁴: **C07C 63/36**, C07C 65/24, C07C 65/26, C07C 69/76, C07C 153/11, C07F 7/18, C07C 43/23, C07C 103/76, C07D 295/18

(54) **Dérivés benzonaphtaléniques, leur procédé de préparation et leur application dans les domaines pharmaceutique et cosmétique.**

(30) Priorité: **11.04.85 LU 85849**

(43) Date de publication de la demande: **29.10.86 Bulletin 86/44**

(45) Mention de la délivrance du brevet: **08.02.89 Bulletin 89/6**

(84) Etats contractants désignés: **AT BE CH DE FR GB IT LI NL SE**

(56) Documents cités: **US-A- 4 454 341**

(73) Titulaire: **CENTRE INTERNATIONAL DE RECHERCHES DERMATOLOGIQUES C.I.R.D. Groupement d'Intérêt Economique dit:, Sophia Antipolis, F-06560 Valbonne(FR)**

(72) Inventeur: **Shroot, Braham, Villa 35 - Hameaux de Val-Bosquet, Chemin de Val-Bosquet F-06600 Antibes(FR)**
Inventeur: **Eustache, Jacques, 42, avenue Alphonse Daudet, F-06130 Grasse(FR)**
Inventeur: **Bernardon, Jean-Michel, 89, Route de Nice Le Rouret, F-06650 Nice(FR)**

(74) Mandataire: **Nony, Michel et al, Cabinet NONY & CIE 29, rue Cambacérès, F-75008 Paris(FR)**

EP 0 199 636 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés benzonaphtaléniques, leur procédé de préparation et leur utilisation dans les domaines thérapeutique et cosmétique.

Ces nouveaux dérivés benzonaphtaléniques trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans le traitement des maladies de dégénérescence du tissu conjonctif ainsi qu'une activité antitumorale. En outre, ces dérivés peuvent être utilisés dans le traitement de l'atopie qu'elle soit cutanée ou respiratoire.

Ils trouvent également une application dans le domaine ophtalmologique notamment pour le traitement des cornéopathies.

Divers composés ont été déjà proposés pour ce type de traitement, notamment les composés connus sous les dénominations de "rétinoïdes" dont les représentants les plus connus sont les acides trans- et cis-rétinoïques (trétinoïne et isotrétinoïne) et l'étrétinate.

Il a également été proposé dans le brevet US 4 454 341 d'utiliser dans le traitement des désordres cutanés notamment de l'acné et du psoriasis des acides naphtyl ou tétrahydronaphtyl naphtoïques substitués ainsi que leurs dérivés.

Par rapport aux composés connus, les dérivés benzonaphtaléniques selon l' 'invention possèdent une activité renforcée et une meilleure stabilité à la lumière et à l'oxygène de l'air.

Les dérivés benzonaphtaléniques selon l'invention peuvent être représentés par la formule générale suivante:

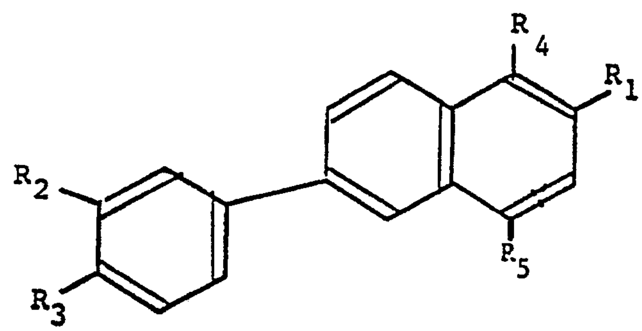

(I)

dans laquelle:

– $R_1$ représente:

$$(i)\ -\underset{\underset{O}{\|}}{C}-R_6 \text{ ou } (ii)\ -CH_2OH$$

– $R_6$ représente un radical

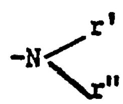

ou un radical $-OR_7$, $R_7$ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono- ou polyhydroxyalkyle, un radical aryle éventuellement substitué ou un reste d'amino-acide ou de sucre aminé ou encore pris ensemble forment un hétérocycle,

– $R_2$ représente un atome d'hydrogène, un radical alkyle, ramifié ou non, ayant de 1 à 15 atomes de carbone, un radical alkoxy ayant de 1 à 4 atomes de carbone, ou un radical cycloaliphatique,

– $R_3$ représente un atome d'hydrogène, un radical hydroxyle, un radical alkyle, ramifié ou non, ayant de 1 à 4 atomes de carbone, un radical alkoxy ayant de 1 à 10 atomes de carbone, un radical cycloaliphatique substitué ou non, un radical thio-cycloaliphatique ou un radical de formule $-O-Si(CH_3)_2-R_8$, $R_8$ représentant un radical alkyle inférieur linéaire ou ramifié,

–$R_4$ et $R_5$, identiques ou différents, un atome d'hydrogène, un radical alkyle inférieur, un radical hydroxyle ou un radical acyloxy inférieur, les sels desdits dérivés benzonaphtaléniques de formule (I).

Par radical alkyle inférieur, on doit entendre les radicaux ayant de 1 à 6 atomes de carbone, notamment les radicaux méthyle, éthyle, isopropyle, butyle et tertiobutyle.

Par radical alkoxy, on doit entendre notamment les radicaux méthoxy, éthoxy, isopropoxy, hexyloxy et décyloxy.

Par radical acyloxy inférieur on doit entendre les radicaux ayant de 1 à 4 atomes de carbone notamment les radicaux acétyloxy et propionyloxy.

Par radical monohydroxyalkyle, on doit entendre un radical ayant 2 ou 3 atomes de carbone, notamment un radical hydroxy-2-éthyle et hydroxy-2-propyle.

Parmi les restes de sucres aminés on peut citer ceux dérivant de glucosamine, de galactosamine et de mannosamine.

Par radical polyhydroxyalkyle, on doit entendre un radical comportant de 3 à 6 atomes de carbone et de 2 à 5 groupes hydroxyles tels que les radicaux dihydroxy-2,3-propyle, dihydroxy-1,3-propyle, ou le reste du pentaérythritol.

Par radical cycloaliphatique, on doit entendre un radical mono ou polycyclique tel que par exemple le radical méthyl-1-cyclohexyle ou le radical 1-adamantyle.

Comme radical thiocycloaliphatique préféré, on peut notamment citer le radical 1-adamantyl-thio.

Lorsque les radicaux r' et r" pris ensemble forment un hétérocycle, celui-ci est de préférence un radical pipéridino, pipérazino, morpholino ou pyrrolidino.

Les composés préférés de formule (I) sont plus particulièrement ceux correspondant à la formule suivante:

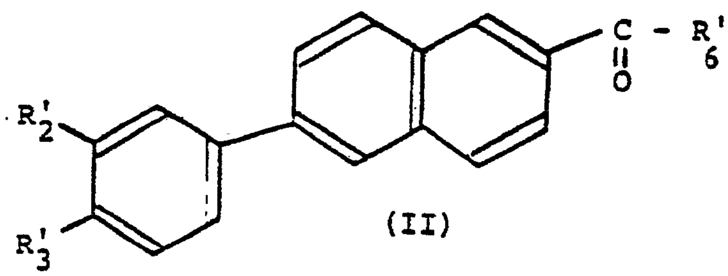

(II)

dans laquelle:
$R'_6$ représente un radical

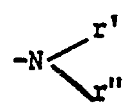

ou un radical $-OR'_7$
r' et r" identiques ou différents, représentent un atome d'hydrogène un radical alkyle inférieur, ou pris ensemble forment un radical morpholino,
$R'_7$ représentant un atome d'hydrogène ou un radical alkyle inférieur,
$R'_2$ représente un atome d'hydrogène, un radical alkyle, un radical alkoxy, ou un radical 1-adamantyle,
et $R'_3$ représente un atome d'hydrogène, un radical hydroxyle, un radical alkyle, un radical alkoxy, ou un radical 1-adamantylthio.

Parmi les composés selon l'invention, on peut notamment citer:
- l'acide 6-(3-méthylphényl)-2-naphtoïque et son ester méthylique,
- l'acide 6-(4-tertiobutyl phényl)-2-naphtoïque et son ester méthylique,
- l'acide 6-(3-tertiobutyl phényl)-2-naphtoïque et son ester méthylique,
- l'acide 6-(3,4-diméthoxy phényl)-2-naphtoïque et son ester méthylique,
- l'acide 6-[p-(1-adamantylthio)phényl]-2-naphtoïque et son ester méthylique,
- l'acide 6-[3-(1-adamantyl)-4-méthoxyphényl]-2-naphtoïque et son ester méthylique.
- L'Ester méthylique de l'acide 6-[3-(1-adamantyl-4-tert-butyldiméthylsilyloxyphényl]-2-naphtoïque,
- L'Ester méthylique de l'acide 6-[3-(1-adamantyl)-4-hydroxyphényl]-2-naphtoïque,
- L'Acide 6-[3-(1-adamantyl)-4-hydroxyphényl]-2-naphtoïque,
- L'Ester méthylique de l'acide 6-[3-(1-adamantyl)-4-décyloxyphényl-2-naphtoïque,
- L'Acide 6-[3-(1-adamantyl)-4-décyloxyphényl]-2-naphtoïque,
- L'Ester méthylique de l'acide 6-[3-(1-adamantyl)-4-hexyloxyphényl]-2-naphtoïque,
- L'Acide 6-[3-(1-adamantyl)-4-hexyloxyphényl]-2-naphtoïque,
- L'Ester méthylique de l'acide 6-[3-(1-adamantyl)-4-méthoxyphényl]-4-acétoxy-1-méthyl-2-naphtoïque,
- L'Acide 6-[3-(1-adamantyl)-4-méthoxyphényl]-4-hydroxy-1-méthyl-2-naptoïque,
- L'Ester méthylique de l'acide 6-[3-(1-adamantyl)-4-méthoxyphényl]-4-hydroxy-1-méthyl-2-naphtoïque,

– L'Ester méthylique de l'acide 6-[3-(1-adamantyl)-4-méthoxyphényl]-1-méthyl-2-naphtoïque,
– L'Acide 6-[3-(1-adamantyl)-4-méthoxyphényl]-1-méthyl-2-naphtoïque,
– le 6-[3-(1-adamantyl)-4-méthoxyphényl]-2-naphtalène méthanol,
– L'Ethylamide de l'acide 6-[3-(1-adamantyl)-4-méthoxyphényl]-2-naphtoïque,
– Le Morpholide de l'acide 6-[3-(1-adamantyl)-4-méthoxyphényl]-2-naphtoïque,
– L'Ester méthylique de l'acide 6-(3-tert-butyl-4-méthoxyphényl)-2-naphtoïque,
– L'Acide 6 (3-tert-butyl-4-méthoxyphényl)-2-naphtoïque,
– L'Ester méthylique de l'acide 6-[3-(1,1-diméthyldécyl)-4-méthoxyphényl]-2-naphtoïque, et
– L'Acide 6-[3-(1,1-diméthyldécyl)-4-méthoxyphényl]-2-naphtoïque.

La présente invention a également pour objet le procédé de préparation des composés de formule (I).

Selon ce procédé les composés de formule (I) sont obtenus par une réaction de couplage entre un composé halogéné de formule (III) et un dérivé halogéné du naphtalène de formule (IV):

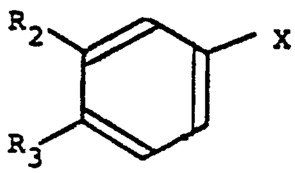

(III)

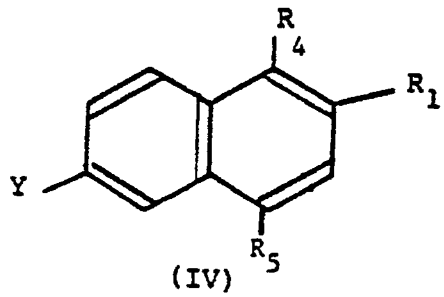

(IV)

dans lesquelles: les radicaux $R_1$ à $R_5$ ont les mêmes significations que celles données ci-dessus pour la formule (I) et
X et Y représentent Cl, Br, F ou I.

Selon ce procédé de couplage, le composé halogéné de formule (III) est transformé en son magnésien, son lithien ou son zincique selon les méthodes connues dans la littérature et est couplé avec le dérivé halogéné du naphtalène de formule (IV) en utilisant, comme catalyseur de réaction, un métal de transition ou l'un de ses complexes.

Comme catalyseur, on peut en particulier mentionner ceux dérivés du nickel ou du palladium et en particulier les composés de $Ni_{II}$ ($NiCl_2$) avec diverses phosphines.

La réaction de couplage est généralement effectuée à une température comprise entre –20 et +30°C dans un solvant anhydre tel que par exemple le diméthylformamide ou le tétrahydrofuranne.

Le produit obtenu peut être purifié par recristallisation ou par chromatographie sur colonne de silice.

Il va de soi que le choix du dérivé halogéné du naphtalène de formule (IV), pour la réaction de couplage avec le composé halogéné de formule (III), doit être tel qu'il puisse conduire par réaction ultérieure aux différentes significations du radical $R_1$.

Lorsque les composés selon l'invention se présentent sous forme de sels, il peut s'agir soit de sels d'un métal alcalin ou alcalino-terreux ou d'une amine organique lorsqu'ils comportent au moins une fonction acide libre.

La présente invention a également pour objet à titre de médicament les composés de formule (I) telle que définie ci-dessus.

Ces composés présentent une excellente activité dans le test d'inhibition de l'ornithine décarboxylase après induction par "tape stripping" chez le rat nu. Ce test est admis comme mesure de l'action des rétinoïdes sur les phénomènes de prolifération cellulaire.

A titre d'indication on peut signaler, que dans ce test, l'acide 6-[3-(1-adamantyl)-4 méthoxyphényl]-2-naphtoique présente une dose efficace 50 comprise entre 5 et 25 nmoles appliquée par $cm^2$.

Les composés selon l'invention présentent également une activité renforcée dans le test de différenciation des cellules de tératocarcinone embryonnaire de souris F9 (Cancer Research 43, page 5268, 1983).

A titre d'illustration, l'acide 6-[3-(1-adamantyl)-4 méthoxyphényl]-2-naphtoïque, à la concentration 0,01 micromolaire, induit la différenciation de cellules de carcinome F9 en cellules d'endoderme.

L'acide 6-(3-tertiobutylphényl)-2 naphtoïque agit de même à la concentration 1 micromolaire.

Par ailleurs, le test d'irritation effectué chez le lapin a montré que les composés de formule I étaient moins irritants que les rétinoïdes connus de structure analogue. Leur toxicité aigue est par ailleurs moins forte.

Ces composés conviennent particulièrement bien pour traiter les affections dermatologiques liées à un désordre de la kératinisation (différenciation, prolifération) ainsi que les affections dermatologiques, ou autres, à composante inflammatoire et/ou immunoallergique notamment:
– les acnés vulgaires, comédoniennes ou polymorphes, les acnés séniles, solaires, et les acnés médicamenteuses ou professionnelles

– les formes étendues et/ou sévères de psoriasis, et les autres troubles de la kératinisation, et notamment les ichtyoses et états ichtyosiformes
– la maladie de Darier
– les kératodermies palmo-plantaires
– les leucoplasies et états leucoplasiformes, le lichen plan
– toutes proliférations dermatologiques bénignes ou malignes, sévères ou étendues.

Ils sont également actifs pour certaines affections rhumatismales notamment le rhumatisme psoriasique, pour les atopies cutanées ou respiratoires ainsi que pour certains problèmes ophtalmologiques relatifs aux cornéopathies.

La présente invention a donc également pour objet des compositions médicamenteuses contenant au moins un composé de formule (I) tel que défini ci-dessus et/ou un de ses sels.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels.

Comme ceci a été précédemment indiqué, les dérivés benzonaphtaléniques selon l'invention présentent, par rapport aux rétinoïdes classiques, une meilleure stabilité à la lumière et à l'oxygène, ceci étant essentiellement dû au fait qu'ils ne possèdent pas de double liaison facilement isomérisable.

Les composés selon l'invention sont généralement administrés à une dose journalière d'environ 2 µg/kg à 2 mg/kg de poids corporel.

Comme support des compositions, on peut utiliser tout support conventionnel, le composé actif se trouvant soit à l'état dissous soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base des composés selon l'invention se présentent sous forme d'onguents, de teintures, de crèmes, de pommades, de poudres, de timbres, de tampons imbibés, de solutions, de lotions, de gels, de sprays ou encore de suspensions.

Ces compositions par voie topique peuvent se présenter soit sous forme anhydre, soit sous forme aqueuse selon l'indication clinique. Par voie oculaire, ce sont principalement des collyres.

Les compositions par voie topique ou oculaire contiennent de préférence entre 0,0005 et 5% en poids de composé actif.

Les composés de formule (I), selon l'invention, trouvent également une application dans le domaine cosmétique, en particulier dans l'hygiène corporelle et capillaire et notamment pour l'acné, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) et/ou un de ses sels, cette composition se présentant notamment sous forme de lotion, gel, savon ou shampooing.

La concentration en composé(s) de formule (I), dans les compositions cosmétiques est comprise entre 0,0005 et 2% en poids et de préférence entre 0,01 et 1% en poids.

Les compositions médicamenteuses et cosmétiques selon l'invention peuvent contenir des additifs inertes ou même pharmacodynamiquement ou cosmétiquement actifs et notamment: des agents hydratants comme la thiamorpholinone et ses dérivés ou l'urée; des agents antiséborrhéiques, tels que la S-carboxyméthylcystéine, la S-benzyl-cystéamine et leurs dérivés, la tioxolone; des antibiotiques comme l'érythromycine, la néomycine ou les tétracyclines; des agents favorisant la repousse des cheveux, comme le "Minoxidil" (2,4-diamino-6-pipéridino-pyrimidine-3-oxyde) et ses dérivés, le Diazoxide et le Phénytoïn; des agents anti-inflammatoires stéroïdiens; des caroténoïdes et, notamment, le β-carotène; des agents anti-psoriasiques tels que l'anthraline et ses dérivés et les acides eicosatétraynoïque-5,8,11,14 et triynoïque-5,8,11.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que l'α -tocophérol, le butylhydroxy-anisole ou le butylhydroxy toluène.

On va maintenant donner, à titre d'illustration et sans aucun caractère limitatif, plusieurs exemples de préparation des composés actifs de formule (I) selon l'invention ainsi que des exemples de compositions les contenant.

EXEMPLE 1

ESTER METHYLIQUE DE L'ACIDE 6-(3-METHYL PHENYL)-2-NAPHTOIQUE composé de formule (II) dans laquelle: $R'_3$ = H et $R'_2$ = $-CH_3$ et $R'_6$ = $-OCH_3$

342 mg (2 mmol) de 3-bromotoluène dans 4 ml de THF sont convertis en magnésien correspondant puis traités par un équivalent de chlorure de zinc pour donner le zincique correspondant. On ajoute successivement 310 mg (1,17 mmol) de 6-bromo-2-naphtoate de méthyle, puis 10 mg (0,02 mmol) de catalyseur $NiCl_2$/1,2-(diphénylphosphino)éthane (DPPE). On agite à température ambiante pendant 30 mn. On enlève les sels minéraux par passage à travers une colonne de silice (2 x 3 cm), puis on évapore à sec. Le résidu est chromatographié (HPLC colonne Zorbax sil), en utilisant comme éluant un mélange de cyclohexane (75%) et d'éther (25%). On recueille ainsi le produit qui a un RF=0,45 (plaque de silice, éluant: hexane 50%, dichlorométhane 50%). Le produit cristallise par évaporation des solvants de chromatographie. Le rendement (Rdt) est de 84%. Point de fusion = 107°C.

EXEMPLE 2

ESTER METHYLIQUE DE L'ACIDE 6-(4-t-BUTYL PHENYL)-2-NAPHTOIQUE composé de formule (II) dans laquelle: $R'_2 = H$, $R'_3 = -C(CH_3)_3$ et $R'_6 = -OCH_3$

De manière analogue à l'exemple 1, en partant de 639 mg (3,0 mmol) de 4-bromo t-butyl benzène et 465 mg (1,75 mmol) de 6-bromo-2-naphtoate de méthyle, on obtient 0,30 g du produit attendu (Rdt: 54%). Point de fusion = 154°C.

EXEMPLE 3

ESTER METHYLIQUE DE L'ACIDE 6-(3-t-BUTYL PHENYL)-2-NAPHTOIQUE composé de formule (II) dans laquelle: $R'_3 = H$, $R'_2 = -C(CH_3)_3$ et $R'_6 = -OCH_3$

On ajoute 3,50 g (16,4 mmole) de 3-tert-butylbromobenzène à une suspension de magnésium (0,44 g, 18 m Atg dans 20 ml de tétrahydrofuranne sec. La réaction est initiée par addition d'un cristal d'iode puis poursuivie à 50°C pendant 30 mn.

On ajoute ensuite 2,46 g (18 mmole) de chlorure de zinc anhydre dissous dans 20 ml de tétrahydrofuranne sec. Après 15 mn, le mélange réactionnel est refroidi à 0°C et on ajoute 3,63 g (13,7 mmole) de 6-bromo-2-naphtoate de méthyle et 86 mg (0,26 mmole) du complexe $NiCl_2$/DPPE.

Après agitation pendant 1h à température ambiante, on ajoute de l'eau (100 ml), et extrait à l'éther. Après lavage de la phase organique avec une solution saturée de bicarbonate de sodium, et à l'eau, puis séchage (sulfate de sodium) et évaporation des solvants, le résidu obtenu est recristallisé dans l'heptane. On obtient ainsi 3,12 g d'ester méthylique de l'acide 6-(3-tert-butylphényl)-2-naphtoïque qui fond à 138°C.

EXEMPLE 4

ACIDE 6-(3-T-BUTYL PHENYL)-2 NAPTHOIQUE composé de formule (II) dans laquelle: $R'_3 = H$, $R'_2 = -C(CH_3)_3$ et $R'_6 = -OH$

1,0 g (3,14 mmole) d'ester méthylique de l'acide 6-(3-tert-butyl-phényl)-2 naphtoïque obtenu à l'exemple 3 sont ajoutés à un mélange d'éthanol 95% (40 ml) et de soude (4 ml, 5 N).

On chauffe à 60°C pendant 2h, puis ajoute 50 ml d'eau, acidifie à pH1 avec de l'acide chlorhydrique 2 N. On extrait à l'éther et la phase organique est ensuite lavée à l'eau jusqu'à neutralité. Après séchage (sulfate de sodium) et évaporation du solvant, on obtient l'acide 6-(tert-butylphényl)-2-naphtoïque (900 mg) qui se sublime à 190°C.

EXEMPLE 5

ESTER METHYLIQUE DE L'ACIDE 6-[p-(1-ADAMANTYLTHIO)PHENYL]-2-NAPHTOIQUE composé de formule (II) dans laquelle: $R'_2 = H$, $R'_3$ = 1-adamantylthio et $R'_6 = -OCH_3$

a) (1-Adamantylthio)-p-bromobenzène

3,78 g (20 mmol) de p-bromothiophénol, 3,04 g (20 mmol) de 1-adamantanol et 10 ml d'acide trifluoracétique sont agités à température ambiante pendant huit heures. On verse ensuite dans l'eau, ajoute du bicarbonate de sodium jusqu'à neutralité, extrait au chlorure de méthylène, séche la phase organique et évapore. Après recristallisation dans l'isooctane on obtient 5,9 g du produit attendu (Rdt: 92%). Point de fusion: 121–122°C.

b) Ester méthylique de l'acide 6-[p-(1-adamantylthio) phényl]-2-naphtoïque

0,64 g (26,5 mAtg) de magnésium mis en suspension dans 10 ml de tétrahydrofuranne (T.H.F) sont traités goutte à goutte par 5,7 g (17,6 mmol) de (1-adamantylthio)-p-bromobenzène. Après chauffage à reflux pendant 2 heures et refroidissement à 20°C on ajoute 2,4 g (17,6 mmol) de $ZnCl_2$ anhydre et agite pendant une heure à 20°C. On ajoute 2,8 g (10,4 mmol) de 6-bromo-2-naphtoate de méthyle puis 92 mg de complexe $NiCl_2$/1,2-(diphénylphosphino)éthane (DPPE).

On agite à température ambiante pendant deux heures, verse dans l'eau et extrait au chlorure de méthylène, lave au bicarbonate de sodium, sèche puis évapore. Le résidu est recristallisé dans un mélange d'oxyde de diisopropyle et d'acétate d'éthyle. On obtient ainsi 3,7 g du produit attendu (Rdt: 84%). Point de fusion: 189–190°C

EXEMPLE 6

ACIDE 6-[p-(1-ADAMANTYLTHIO)PHENYL]-2-NAPHTOIQUE composé de formule (II) dans laquelle: $R'_2$ = H, $R'_6$ = –OH et $R'_3$ = 1-adamantylthio

3 g (7 mmol) de l'ester obtenu à l'exemple 5 (b) sont traités par une solution de soude dans le méthanol (150 ml, 2N). On chauffe à reflux pendant 12 h, évapore, reprend par l'eau et acidifie avec de l'acide chlorhydrique concentré. On filtre le solide obtenu et séche sous vide sur anhydride phosphorique. On triture le solide blanc, dans du méthanol au reflux, refroidit et filtre. On obtient ainsi 2,5 g (Rdt: 86%) du produit attendu. Point de fusion: 334–336°C

EXEMPLE 7

ESTER METHYLIQUE DE L'ACIDE 6-(3-4-DIMETHOXYPHENYL)-2-NAPHTOIQUE composé de formule (II) dans laquelle: $R'_2$ = $R'_3$ = $R'_6$ = $-OCH_3$

0,93 g (38,3 mAtg) de magnésium dans 20 ml de THF sont traités goutte à goutte par 5,5 g (25,5 mmol) de 4-bromovératrole. L'addition étant terminée, on chauffe à reflux pendant deux heures. On refroidit et ajoute 3,48 g (25,5 mmol) de $ZnCl_2$ anhydre et agite une heure à température ambiante. On ajoute ensuite 3,98 g (15 mmol) de 6-bromo-2-naphtoate de méthyle puis 130 mg de complexe $NiCl_2$/DPPE et agite deux heures à température ambiante. On verse dans l'eau, extrait au dichlorométhane, sèche la phase organique et évapore. Le résidu est recristallisé dans un mélange d'éther isopropylique et d'acétate d'éthyle. On obtient ainsi 3,4 g du produit attendu (Rdt: 70%). Point de fusion: 147–148°C.

EXEMPLE 8

ACIDE 6-(3,4-DIMETHOXYPHENYL)-2-NAPHTOIQUE composé de formule (II) dans laquelle $R'_2$ = $R'_3$ = $-OCH_3$ et $R'_6$ = –OH

2,6 g (8 mmol) de l'ester obtenu à l'exemple 7 sont traités par une solution de soude dans le méthanol (200 ml, 2N). On chauffe à reflux durant 8 heures, évapore, reprend par l'eau, acidifie avec HCl concentré, et filtre. Le solide ainsi obtenu est séché sous vide (sur $P_2O_5$). On triture le solide blanc dans le méthanol à reflux. Après refroidissement on filtre et obtient 2,3 g de produit attendu (Rdt: 92%). Point de fusion: 241–243°C.

EXEMPLE 9

ESTER METHYLIQUE DE L'ACIDE 6-[3-(1-ADAMANTYL)-4-METHOXYPHENYL]-2-NAPHTOIQUE composé de formule (II) dans laquelle: $R'_3$ = $-OCH_3$ $R'_2$ = 1-adamantyl et $R'_6$ = $-OCH_3$

a) 2-(1-Adamantyl)-4-bromophénol

34,6 g (200 mmol) de p-bromophénol et 30,4 g (200 mmol) de 1-amandantanol sont dissous dans 100 ml de dichlorométhane. On ajoute goutte à goutte 10 ml d'acide sulfurique concentré et agite huit heures à température ambiante. On verse dans l'eau, neutralise par du bicarbonate de sodium, extrait au chlorure de méthylène, sèche et évapore. Après recristallisation dans l'isooctane on obtient 52,8 g (Rdt: 86%) du composé attendu. Point de fusion: 140–141°C.

b) 2-(1-adamantyl)-4-bromoanisole

A une suspension d'hydrure de sodium (80% dans l'huile, 4,32 g, 144 mmol) dans 50 ml de THF, on ajoute goutte à goutte, en maintenant la température à 20°C, 36,8 g (120 mmol) de 2-(1-adamantyl)-4-bromophénol et agite 1 h à température ambiante. On ajoute ensuite 9 ml (144 mmol) d'iodure de méthyle et agite encore 2 h à 20°C. On verse dans l'eau, extrait à l'éther, sèche et évapore. Le produit est purifié par passage sur une colonne de silice (10 × 30 cm) en éluant avec un mélange d'hexane (90%) et de dichlorométhane (10%). Par évaporation, on obtient 26,2 g d'un solide blanc (Rdt: 68%). Point de fusion: 138–139°C.

c) Ester méthylique de l'acide 6-[-3-(1-adamantyl)-4-methoxyphényl]-2-naphtoïque

A une suspension de magnésium (1,64 g, 67,5 mAtg) dans 30 ml de THF, on ajoute une solution de 1,4 g (4,5 mmol) de 2-(1-adamantyl)-4-bromoanisole et 0,39 ml de dibromoéthane dans 10 ml de THF. On agite jusqu'à ce que la réaction soit initiée puis ajoute goutte à goutte une solution de 13,1 g (40,8 mmol) de 2-(1-adamantyl)-4-bromoanisole dans 90 ml de THF puis chauffe à reflux pendant 2 h. On refroidit à 20°C et ajoute 6,2 g (45 mmol) de $ZnCl_2$ anhydre. On agite 1 h à 20°C et ajoute 7,95 g (30 mmol) de 6-bromo-2-naphtoate de méthyle, puis 300 g du complexe $NiCl_2$/DPPE. On agite encore 2 h à 20°C, verse dans l'eau, extrait avec $CH_2Cl_2$, sèche, évapore. Le produit est isolé par chromatographie sur colonne, en éluant avec un mélange d'heptane (70%) et de dichlorométane (30%) puis par recristallisation dans l'acétate d'éthyle. On obtient ainsi 12,2 g du produit attendu (Rdt: 78%). Point de fusion: 222–223°C.

EXEMPLE 10

ACIDE 6-[3-(1-ADAMANTYL)-4-METHOXYPHENYL]-2-NAPHTOIQUE composé de formule (II) dans laquelle: $R'_3 = -OCH_3$, $R'_2$ = 1-adamantyl et $R'_6 = -OH$

10,5 g de l'ester obtenu à l'exemple 9 (c) sont traités par une solution de soude dans le méthanol (200 ml, 4,2 N). On chauffe à reflux pendant 48 h, évapore les solvants, reprend par l'eau et acidifie avec de l'acide chlorhydrique concentré, filtre le solide et sèche sous vide sur anhydride phosphorique.

Le solide blanc obtenue est recristallisé dans un mélange de THF et d'acétate d'éthyle. On obtient ainsi 8,2 g (Rdt: 81%) du produit attendu. Point de fusion: 325–327°C.

EXEMPLE 11

ESTER METHYLIQUE DE L'ACIDE 6-[3-(1-ADAMANTYL)-4-TERT-BUTYLDIMETHYLSILYLOXYPHENYL]-2-NAPHTOIQUE
composé de formule (I) dans laquell: $R_4 = R_5 = H$, $R_2$ = 1-adamantyle,

$$R_3 = -OSi(CH_3)_2C_3H_7 \text{ et } R_1 = -\underset{\underset{O}{\|}}{C}\ OCH_3$$

a) 2-(1-adamantyl)-4-bromo-1-tert-butyldiméthylsilyloxybenzène.

30,7 g de 2-adamantyl-4-bromophénol (100 mmol), sont dissous dans du DMF (200 ml). On ajoute de la triéthylamine (15,4 ml, 110 mmol), et de la 4-N,N-diméthylaminopyridine (DMAP, 500 mg, 4 mmol).

A la solution ainsi obtenue, on ajoute goutte à goutte une solution de chlorure de tert-butyldiméthylsilyle [15,7 g, 104 mmol dans du DMF (100 ml)]. On agite à température ambiante pendant 4h, verse dans l'eau, extrait à l'éther, sèche ($MgSO_4$) et évapore. Le résidu est dissous dans l'hexane et purifié par passage sur une colonne de silice (éluant: hexane). On obtient ainsi 36,2 g (86%) de 2-adamantyl-4-bromo-1-tert-butyldiméthylsilyloxybenzène. Point de fusion: 111°C.

b) Ester méthylique de l'acide 6-[3-(1-adamantyl)-4-tert-butyldiméthylsilyloxyphényl] -2-naphtoïque.

33,3 g (79 mmol) du composé ci-dessus, dissous dans 200 ml de THF sont ajoutés lentement à une suspension de magnésium (2,9 g, 118 Atg) dans 60 ml de THF. Une fois l'addition terminée, on chauffe à reflux pendant 2 h. On laisse revenir à température ambiante et ajoute 10,8 g (79 mmol) de chlorure de zinc anhydre. On agite une heure à température ambiante et ajoute 10,5 g (39,5 mmol) de 6-bromo-2-naphtoate de méthyle et 500 mg du complexe $NiCl_2$/DPPE. On agite encore 2 h à température ambiante, verse dans l'eau, extrait avec $CH_2Cl_2$, sèche et évapore. Le résidu est chromatographié sur colonne de silice [éluant: mélange d'heptane (70%) et d'éther (30%)]. On obtient ainsi 18,5 g (90%) d'ester méthylique de l'acide 6-[3-(1-adamantyl)-4-tert-butyldiméthylsilyloxyphényl[-2-naphtoïque. Point de fusion: 152–153°C.

EXEMPLE 12

ESTER METHYLIQUE DE L'ACIDE 6-[3-(1-ADAMANTYL)-4-HYDROXYPHENYL] -2-NAPHTOIQUE
composé de formule (I) dans laquelle: $R_4 = R_5 = H$, $R_2$ = 1-adamantyle $R_3 = -OH$ et $R_1 = -COOCH_3$

17,5 g (33 mmol) de l'ester obtenu à l'exemple 11 sont dissous dans 300 ml de THF. On ajoute goutte à goutte 36,6 ml d'une solution molaire de fluorure de tétrabutylammonium dans le THF. On agite 2 h à température ambiante, jette dans l'eau, extrait avec $CH_2Cl_2$. La phase organique est recueillie, séchée ($MgSO_4$), et les solvants évaporés. Le résidu obtenu est recristallisé dans un mélange d'acétate d'ethyle (70%) et de THF (30%) pour donner l'ester attendu: 11 g (81%). Point de fusion: 266°C.

EXEMPLE 13

ACIDE 6-[3-(1-ADAMANTYL)-4-HYDROXYPHENYL]-2-NAPHTOIQUE composé de formule (I) dans laquelle: $R_4 = R_5 = H$, $R_2$ = 1-adamantyl $R_3 = OH$ et $R_1 = -COOH$

5 g (12 mmol) de l'ester obtenu à l'exemple 12 sont traités par 200 ml de soude méthanolique (2 N), sous azote, pendant 8 h. On évapore les solvants, reprend par l'eau et acidifie à pH 1 (HCl concentré). On filtre, lave à l'eau et extrait le produit solide avec de l'éther éthylique. On sèche ($MgSO_4$), et évapore. Le résidu est recristallisé dans l'éther isopropylique. On obtient ainsi 3,8 g (79%) de l'acide attendu. Point de fusion: 270–271°C.

EXEMPLE 14

ESTER METHYLIQUE DE L'ACIDE 6-[3-(1-ADAMANTYL)-4-DECYLOXYPHENYL]-2-NAPTHOIQUE composé de formule (I) dans laquelle: $R_4 = R_5 = H$, $R_2$ = 1-adamantyle, $R_3 = -OC_{10}H_{21}$ et $R_1 = -CO_2CH_3$

a) 2-(1-adamantyl)-4-bromo-1-décyloxybenzène.

A une suspension d'hydrure de sodium (80% dans l'huile, 3,2 g, 104 mmol) dans 100 ml de THF, on ajoute goutte à goutte une solution de 2-(1-adamantyl)-4-bromophénol (29 g, 95 mmol) dans 200 ml de THF. On agite jusqu'à cessation du dégagement gazeux puis ajoute 27,8 g (23 ml, 104 mmol) de 1-iododécane et 100 ml de DMF. On agite 12 h à température ambiante, verse dans l'eau, extrait à l'éther, sèche et évapore les solvants. Le résidu obtenu est purifié par passage sur colonne de silice (éluant: heptane). On obtient ainsi 40,7 g (96%) de 2-(1-adamantyl)-4-bromo-1-décyloxybenzène. Point de fusion: 69–70°C.

b) Ester méthylique de l'acide 6-[3-(1-adamantyl)-4-décyloxyphényl]-2-maphtoïque.

De manière analogue à l'exemple 9c, à partir de 17,9 g (40 mmol) du dérivé bromé obtenu ci-dessus, et de 5,3 g de 6-bromo-2-naphtoate de méthyle, on obtient 7,4 g (67%) de l'ester attendu. Point de fusion: 113–114°C.

EXEMPLE 15

ACIDE 6-[3-(1-ADAMANTYL)-4-DECYLOXYPHENYL]-2-NAPTHTOIQUE composé de formule (I) dans laquelle: $R_4 = R_5 = H$, $R_2$ = 1-adamantyle, $R_3 = -OC_{10}H_{21}$ et $R_1 = -COOH$

6,3 g (11 mmol) de l'ester obtenu à l'exemple 14 dissous dans 200 ml de THF sont traités à reflux par 200 ml de soude méthanolique 2 M, pendant 4 h. On évapore les solvants, reprend par l'eau, acidifie à pH = 1 (HCl concentré), filtre, lave à l'eau et extrait le solide à l'éther. On sèche et évapore. Le résidu ainsi obtenue est traité par 700ml d'acétate d'éthyle, au reflux. Par refroidissement, on obtient 5,9 g (97%) de l'acide attendu. Point de fusion: 214–215°C.

EXEMPLE 16

ESTER METHYLIQUE DE L'ACIDE 6-[3-(1-ADAMANTYL)-4-HEXYLOXYPHENYL]-2-NAPHTOIQUE composé de formule (I) dans laquelle: $R_4 = R_5 = H$, $R_2$ = 1-adamantyle, $R_3 = -OC_6H_{13}$ et $R_1 = -COOCH_3$

5,3 g (13 mmol) de l'ester obtenu à l'exemple 12 sont dissous dans 100 ml de DMF et ajoutés à une suspension de NaH (80% dans l'huile; 0,46 g; 15,4 mmol) dans le DMF (50 ml). On agite à température ambiante jusqu'à ce que le dégagement gazeux soit terminé puis ajoute du 1-iodohexane (3,26 g; 2,3 ml; 15,4 mmol). On agite 4 h à température ambiante, verse dans l'eau, extrait à l'éther, sèche et évapore. Le résidu est purifié par passage sur colonne de silice [éluant: mélange de dichlorométhane (50%) et d'hexane (50%)] puis recristallisé dans l'isooctane pour donner 5,5 g (87%) de produit pur attendu. Point de fusion: 129–130°C.

EXEMPLE 17

ACIDE 6-[3-(1-ADAMANTYL)-4-HEXYLOXYPHENYL]-2-NAPHTOIQUE composé de formule (I) dans laquelle: $R_4 = R_5 = H$, $R_2$ = 1-adamantyle, $R_3 = -OC_6H_{13}$ et $R_1 = -COOH$

De manière analogue à l'exemple 15, à partir de 4,2 g (8,4 mmol) d'ester obtenu à l'exemple 16, on obtient 3,8 g (95%) d'acide 6-[3-(1-adamantyl)-4-hexyloxyphényl]-2-naphtoïque. Point de fusion: 260–261°C.

EXEMPLE 18

ESTER METHYLIQUE DE L'ACIDE 6-[3-(1-ADAMANTYL)-4-METHOXYPHENYL-4-ACETOXY-1-METHYL-2-NAPHTOIQUE composé de formule (I) dans laquelle: $R_4 = -CH_3$ , $R_5 = -OCOCH_3$, $R_2$ = -1-adamantyle, $R_3 = -OCH_3$ et $R_1 = -CO_2CH_3$

47,6 g (148 mmol) de 2-(1-adamantyl)-4-bromoanisole et 13,9 g (6,3 ml, 74 mmol) de dibromoéthane, dissous dans 1 litre de THF sont ajoutés lentement à une suspension de magnésium (5,4 g, 222 mmol) dans le THF (100 ml). On porte au reflux le mélange pendant 2 h, ajoute du chlorure de zinc (20,2 g, 148 mmol), agite pendant 1 h et ajoute successivement 24,9 g (74 mmol) de 4-acétoxy-6-bromo-1-méthyl-2-naphtoate de méthyle et 500 mg de complexe $NiCl_2$/DPPE. On agite 8h à température ambiante, verse dans une solution aqueuse saturée de chlorure d'ammonium. On extrait avec $CH_2Cl_2$, sèche et évapore les solvants. Le résidu obtenu est purifé par passage sur une colonne de silice (éluant: mélange hexane (40%) et $CH_2Cl_2$ (60%). Le produit obtenu est recristallisé dans l'éther isopropylique. On obtient ainsi 23,5 g (64%) de l'ester attendu. Point de fusion : 201–202°C.

EXEMPLE 19

ACIDE 6-[3-(1-ADAMANTYL)-4-METHOXYPHENYL]-4-HYDROXY-1-METHYL-2-NAPHTOIQUE composé de formule (I) dans laquelle: $R_4 = -CH_3$ , $R_5$ = OH, $R_2$ = 1-adamantyle, $R_3 = -OCH_3$ et $R_1 = -COOH$

23 g (46 mmol) d'ester obtenu à l'exemple 18 sont traités à reflux pendant 12 h par 300 ml de soude méthanolique (2 N). On évapore les solvants, reprend par l'eau et acidifie à pH 1 (HCl concentré). Le solide est filtré, lavé à l'eau, puis dissout dans l'éther éthylique. On sèche ($MgSO_4$), et évapore. Le résidu obtenu est recristallisé dans l'acétate d'éthyle pour donner 18,7 g (92%) de l'acide attendu. Point de fusion: 281–283°C.

EXEMPLE 20

ESTER METHYLIQUE DE L'ACIDE 6-[3-(1-ADAMANTYL)-4-METHOXYPHENYL]-4-HYDROXY-1-METHYL-2-NAPHTOIQUE composé de formule (I) dans laquelle: $R_4 = -CH_3$ , $R_5$ = OH, $R_2$ = 1-adamantyle, $R_3 = -OCH_3$ et $R_1 = -COOCH_3$

17 g (38 mmol) de l'acide obtenu à l'exemple 19 sont traités pendant 12 h à reflux par du méthanol (200 ml), contenant 2 ml d'acide sulfurique. On évapore les solvants, reprend par l'eau, extrait à l'éther, sèche et évapore. Le résidu est purifié par passage sur colonne de silice avec comme éluant un mélange éther (90%)/THF (10%). On recristallise dans l'acétate d'éthyle pour obtenir l'ester attendu pur: 15 g (86%). Point de fusion: 272–274°C.

EXEMPLE 21

ESTER METHYLIOUE DE L'ACIDE 6-[3-(1-ADAMANTYL)-4-METHOXYPHENYL]-1-METHYL-2-NAPHTOIQUE composé de formule (I) dans laquelle: $R_4 = -CH_3$ , $R_5$ = H , $R_2$ = 1-adamantyle, $R_3 = -OCH_3$ et $R_1 = -COOCH_3$

a) 6-[3-(1-adamantyl)-4-méthoxyphényl]-4 diméthylaminothiocarbonyloxy-1-méthyl-2-naphtoate de méthyle.

4,56 g d'ester obtenu à l'exemple 20, dissous dans le THF (100 ml) sont ajoutés goutte à goutte à une suspension d'hydrure de sodium (80% dans l'huile; 360 mg; 12 mmol) dans le DMF (50 ml). On agite 1 h à température ambiante, puis 1 h à 40°C. On ajoute ensuite 1,75 g (14 mmol) de chlorure de diméthylthio carbamoyle, et agite à température ambiante durant 2 h, puis à 40°C durant 2 h. On verse dans l'eau, extrait à l'éther, sèche, et évapore les solvants. Le produit est purifié par passage sur colonne de silice (éluant: $CH_2Cl_2$). On obtient ainsi 4 g (74%) de l'intermédiaire attendu. Point de fusion: 137–138°C.

b) 6-[3-(-1-adamantyl)-4-méthoxyphényl]-4-diméthyl-carbonylthio-1-méthyl-2-naphtoate de méthyle.

3,8 g (7 mmol) de l'ester obtenu ci-dessus sont chauffés sous azote à 260°C durant 0,5 h. Le résidu est repris par du chlorure de méthylène et purifié par passage sur colonne de silice (éluant: $CH_2Cl_2$). La gomme obtenue est reprise par de l'éther isopropylique. On obtient 3,3 g (87%) de l'intermédiaire désiré. Point de fusion: 201–202°C.

c) Ester méthylique de l'acide 6-[3-(1-adamantyl)-4-méthoxyphényl]-1-méthyl-2-naphtoïque.

L'intermédiaire ci-dessus (11 g, 20 mmol) est dissout dans 500 ml d'éthanol. On ajoute 20 g de Nickel de Raney et chauffe à reflux pendant 4 h. On ajoute encore 20 g de Nickel et chauffe encore 1 h. On refroidit, concentre et reprend par $CH_2Cl_2$ (1 litre). On filtre le précipité, recueille le filtrat, sèche et évapore. Le produit est purifié par passage sur colonne de silice (éluant: $CH_2Cl_2$), et recristallisation dans un mélange d'acétate d'éthyle (90%) et de THF (10%). On obtient ainsi 8 g (90%) d'ester méthylique de l'acide 6-[3-(1-adamantyl)-4-méthoxyphényl]-1-méthyl-2-naphtoïque. Point de fusion: 238–239°C.

EXEMPLE 22

ACIDE 6-[3-(1-ADAMANTYL)-4-METHOXYPHENYL]-1-METHYL-2-NAPHTOIQUE composé de formule (I) dans laquelle: $R_4 = -CH_3$, $R_5 = H$, $R_2$ = 1-adamantyle, $R_3 = -OCH_3$ et $R_1 = -COOH$

6,8 g (15,4 mmol) d'ester obtenu à l'exemple 21c sont traités comme à l'exemple 10 pour donner 5,8 g (88%) de l'acide correspondant. Point de fusion: 300–302°C.

EXEMPLE 23

6-[3-(1-ADAMANTYL)4-METHOXYPHENYL]-2-NAPHTALENE METHANOL composé de formule (I) dans laquelle: $R_4 = R_5 = H$, $R_2$ = 1-adamantyle, $R_3 = -OCH_3$ et $R_1 = -CH_2OH$

1,3 g (3 mmol) d'ester obtenu à l'exemple 9 dissous dans le THF (5 ml), sont traités par 171 mg (4,5 mmol) de $LiAlH_4$. On chauffe à reflux, refroidit, traite par une solution aqueuse saturée de tartrate double de sodium et potassium. On filtre, évapore à sec, et recristallise le résidu dans le cyclohexane. On obtient 1,0 g (83%) de 6-[3-(1-adamantyl)-4-méthoxyphényl]-2-naphtalène méthanol. Point de fusion: 163–164°C.

EXEMPLE 24

ETHYLAMIDE DE L'ACIDE 6-[3-(1-ADAMANTYL)-4-METHOXYPHENYL]-2-NAPHTOIQUE composé de formule I dans laquelle: $R_4 = R_5 = H$, $R_2$ = 1-adamantyle, $R_3 = -OCH_3$ et $R_1 = -CONCH_2H_5$

a) Chlorure de l'acide 6-[3-(1-adamantyl)-4-méthoxyphényl]-2-naphtoïque.

4,75 g (1,15 mmol) de l'acide obtenu à l'exemple 10 dans 200 ml de dichlorométhane sont traités par 2,08 g (2,3ml; 1,15 mmol) de dicyclohexamine. On agite à température ambiante jusqu'à dissolution. On évapore les solvants, reprend par de l'éther et filtre le solide ainsi formé (6,8 g). On reprend alors dans le chlorure de méthylène (50 ml) et ajoute 1,37 g (0,84 ml, 1,15 mmol) de chlorure de thionyle. On filtre le sel formé recueille le filtrat, évapore et sèche. On obtient ainsi un solide (3,9 g) qui est utilisé tel quel pour l'étape suivante.

b) Ethylamide de l'acide 6-[3-(1-adamantyl)-4-méthoxy phényl]-2-naphtoïque.

1,3 g (3 mmol) du chlorure d'acide sont dissous dans 20ml de THF. On ajoute 405 mg (600 ml, 9 mmol) d'éthylamine et agite 2 h à température ambiante. On verse dans l'eau, extrait ($CH_2Cl_2$) sèche et évapore. Le résidu est recristallisé dans l'acétate d'éthyle. On obtient ainsi 1,1 g (85%) d'amide. Point de fusion: 220–221°C.

EXEMPLE 25

MORPHOLIDE DE L'ACIDE 6-[3-(1-ADAMANTYL)-4-METHOXYPHENYL]-2-NAPTHTOIQUE

De manière analogue à l'exemple 24, à partir de 1,3 g de chlorure d'acide et de 780 mg (780ml; 9 mmol) de morpholine, on obtient 1,3 g (91%) du morpholide. Point de fusion: 212–213°C.

EXEMPLE 26

ESTER METHYLIQUE DE L'ACIDE 6-[3-TERT-BUTYL-4-METHOXYPHENYL]-2-NAPHTOIQUE composé de formule (II) dans laquelle: $R'_2$ = tert-butyle, $R'_3 = R'_6 = -OCH_3$

a) 4-bromo-2-tertbutylanisole.

On ajoute en une fois 3,01 g (22,6 mmol) de chlorure d'aluminium à un mélange de 63,5 g (339 mmol) de p-bromoanisole et 31,4 g (339 mmol) de chlorure de tert-butyle. On agite à température ambiante jusqu'à ce que le dégagement gazeux ait cessé (environ 15 minutes), puis à 80°C pendant 15 mn. Le mélange est versé dans la glace, ajoute 300 ml d'eau et extrait à l'éther.

La phase organique est séchée ($MgSO_4$), les solvants évaporés et le résidu purifié par chromatographie sur colonne de silice (éluant: mélange chlorure de méthylène (10%) et d'hexane (90%). On obtient, après évaporation des solvants le 4-bromo-2-tert-butylanisole, sous la forme d'une huile incolore qui cristallise à froid: 31,9 g (39%).

b) Ester méthylique de l'acide 6-[3-tert-butyl-4-méthoxyphényl]-2-naphtoïque.

On ajoute lentement, goutte à goutte une solution de 18,8 g (77 mmol) de 4-bromo-2-tert-butylanisole à 2,26 g (93 mmol) de magnésium en tournures et un cristal d'iode. On chauffe jusqu'à ce que le GRIGNARD commence à se former, puis verse le reste de la solution contenant le dérivé bromé de manière à entretenir un reflux régulier. Une fois l'additon terminée, on chauffe à 40°C pendant 30 minutes, dilue par 200 ml de THF, et refroidit à température ambiante. On ajoute alors 12,7 g (93 mmol) de chlorure de zinc sec en solution dans 20 ml de THF. On agite pendant 30 minutes à température ambiante puis ajoute successivement 12,1 g (46 mmol) de 6-bromo-2-naphtoate de méthyle et 300 mg de complexe $NiCl_2$/DPPE.

Le mélange est agité pendant 10 h à température ambiante, on ajoute ensuite 300 ml d'eau, le THF est évaporé, puis on extrait au chlorure de méthylène. La phase organique est séchée ($MgSO_4$), filtrée, évaporée et purifiée par passage sur une colonne de silice (éluant: mélange de dichlorométhane 50% et d'hexane 50%). Après évaporation des solvants, le residu obtenu est recristallisé dans l'hexane, pour donner l'ester attendu: 11,5 g (72%). Point de fusion: 160°C.

EXEMPLE 27

ACIDE 6-(3-TERT-BUTYL-4-METHOXYPHENYL)-2-NAPHTOIQUE composé de formule (II) dans laquelle: $R'_2$ = tert-butyle, $R'_3 = -OCH_3$ et $R'_6 = -OH$

De manière analogue à l'exemple 15, à partir de 7,0 g (20 mmol) d'ester obtenu à l'exemple 26, on obtient 6,0 g (90%) de l'acide attendu. Point de fusion: 268°C.

EXEMPLE 28

ESTER METHYLIQUE DE L'ACIDE 6-[3-(1,1-DIMETHYLDECYL)-4-METHOXYPHENYL]-2-NAPHTOIQUE composé de formule (II) dans laquelle: $R_4 = R_5 = H$, $R_2 = -C(CH_3)_2C_9H_{19}$,$R_3 = -OCH_3$ et $R_1 = -COOCH_3$

Une solution de 16 g (45 mmol) 2-(1,1-diméthyldécyl)-4-bromo anisole dans 60 ml de THF est additionnée goutte à goutte à 1,3 g (54 mmol) de magnésium et un cristal d'iode. On chauffe légèrement en début d'addition jusqu'à ce que la réaction de formation du GRIGNARD soit initiée, puis le reste de la solution contenant le dérivé bromé est ajouté de manière à entretenir un reflux régulier. Une fois l'addition terminée, on agite 30 minutes à 50°C puis refroidit à température ambiante. On ajoute 7,4 g (54 mmol) de chlorure de zinc en solution dans 50 ml de THF. On agite 30 minutes à temprérature ambiante puis ajoute 6,6 g (25 mmol) de 6-bromo-2-naphtoate de méthyle puis 175 mg de complexe $NiCl_2$/DPPE. On agite 3 h à température ambiante, ajoute 250 ml d'eau et évapore le THF sous pression réduite. On extrait au dichlorométhane, sèche et évapore le solvant. Le résidu est purifié par passage sur une colonne de silice [éluant: mélange de dichlorométhane (60%) et d'hexane (40%)]. Par évaporation, on obtient un solide qui est recristallisé deux fois dans l'hexane pour donner l'ester méthylique de l'acide 6-[3-(1,1-diméthyldécyl)-4-méthoxyphényl]-2-naphtoïque: 7,05 g (61%). Point de fusion: 92°C.

EXEMPLE 29

ACIDE 6-[3-(1,1-DIMETHYLDECYL)-4-METHOXYPHENYL]-2-NAPHTOIQUE composé de formule (II) dans laquelle: $R_4 = R_5 = H$, $R_2 = -C(CH_3)_2C_9H_{19}$, $R_3 = -OCH_3$ et $R_1 = -COOH$

De manière analogue à l'exemple 15, à partir de 3,6 g de l'ester obtenu à l'exemple 28, on obtient 3 g (87%) d'acide 6-[-3(1,1-diméthyldécyl)-4-méthoxyphényl]-2-naphtoïque. Point de fusion: 180°C.

| 1) Crème grasse où le principe actif est en suspension | |
|---|---|
| – Acide 6-[3-(1-adamantyl)-4-methoxyphényl]-2-naphtoïque | 0,001 g |
| – Combinaison d'émulsionnants E/H non ioniques et de corps gras d'origine minérale vendue par la Société Goldschmidt sous le nom de Protegin X | 25,00 g |
| – Huile de vaseline | 10,00 g |
| – Conservateurs q.s. | |
| – Eau QSP | 100,00 g |

Dans cet exemple, le composé actif peut être remplacé par la même quantité de l'acide 6-[3-(1-adamantyl)4-méthoxyphényl]-1-méthyl-2-naphtoïque.

| 2) Crème pour la peau<br>Crème fluide où le principe actif est en suspension | |
|---|---|
| – Ester méthylique de l'acide 6-(4-t-butyl phényl)-2-naphtoïque | 0,02 g |
| – Stéarate de sorbitane polyoxyéthyléné à 20 moles d'oxyde d'éthylène vendu par la Société Atlas sous le nom de Tween 60 | 5,00 g |
| – Monostéarate de sorbitane vendu par la Société Atlas sous le nom de Span 60 | 2,00 g |
| – Alcool cétylique | 5,00 g |
| – Triglycérides des acides caprique et caprylique vendus par la Société Dynamit Nobel sous le nom Miglyol 812 | 10,00 g |
| – Conservateurs q.s. | |
| – Eau QSP | 100,00 g |

| 3) Gel pour la peau ou le cuir chevelu où le principe actif est en suspension | |
|---|---|
| – Ester méthylique de l'acide 6-(4-t-butyl phényl)-2-naphtoïque | 0,10 g |
| – Ethanol | 20,00 g |
| – Hydroxypropylcellulose vendue par la Société Hercules sous le nom de Klucel HF | 2,00 g |
| – Conservateurs q.s. | |
| – Eau QSP | 100,00 g |

| 4) Lotion pour la peau | |
|---|---|
| – Acide 6-[3-(1-adamantyl)-4-méthoxyphényl]-1-méthyl-2-naphtoïque | 0,1 g |
| – Polyéthylèneglycol 400 | 70,0 g |
| – Ethanol | 29,9 g |

Dans cet exemple, le composé actif peut être remplacé par la même quantité de l'acide 6-[3-(1-adamantyl)-4-méthoxyphényl]-2-naphtoïque.

| 5) Onguent | |
|---|---|
| – Acide 6-[3-(1-adamantyl)-4-méthoxyphényl]-2-naphtoïque | 0,001 g |
| – Lanoline | 50 g |
| – Vaseline q.s.p. | 100 g |

<u>PAR VOIE ORALE</u>

| 6) Gelule de 0,30 g | |
|---|---|
| – Acide 6-[3-(1-adamantyl)-4-méthoxyphényl]-2-naphtoïque | 0,003 g |
| – Amidon de maïs | 0,060 g |
| – Lactose QSP | 0,300 g |

La poudre obtenue est conditionnée dans une gelule dont la paroi est composée de gélatine, de $TiO_2$ et d'un conservateur.

7) Capsule de 0,4 g contenant une suspension

| | |
|---|---|
| – Ethylamide de l'acide 6-[3-(1-adamantyl)-4-méthoxyphényl]-2-naphtoïque | 0,005 g |
| – Glycérine | 0,200 g |
| – Saccharose | 0,050 g |
| – Polyéthylène glycol 400 | 0,050 g |
| – Eau purifiée q.s.p. | 0,400 g |

Cette suspension est conditionnée dans une capsule composée de gélatine, glycérine, dioxyde de titane et eau.

**Revendications pour les Etats contractants: BE, CH, DE, FR, GB, IT, LI, NL, SE**

1. Composés benzonaphtaléniques caractérisés par le fait qu'ils répondent à la formule générale suivante:

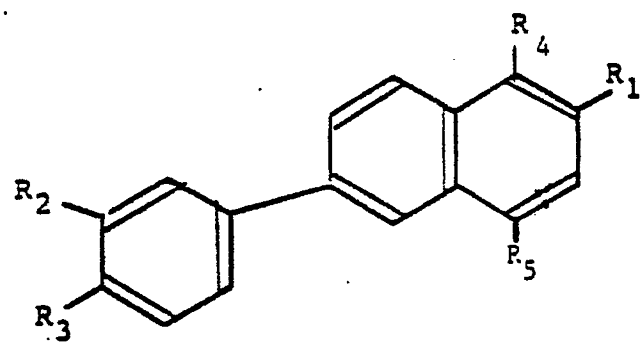

(I)

dans laquelle:
-$R_1$ représente:

$$(i)\ -\underset{\underset{O}{\|}}{C}-R_6 \text{ ou } (ii)\ -CH_2OH$$

$R_6$ représente un radical

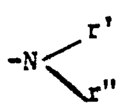

ou un radical $-R_7$, $R_7$ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono ou polyhydroxyalkyle, un radical aryle éventuellement substitué ou un reste d'amino-acide ou de sucre aminé ou encore pris ensemble forment un hétérocycle,
– $R_2$ représente un atome d'hydrogène, un radical alkyle, ramifié ou non, ayant de 1 à 15 atomes de carbone, un radical alkoxy ayant de 1 à 4 atomes de carbone, ou un radical cycloaliphatique,
– $R_3$ représente un atome d'hydrogène, un radical hydroxyle, un radical alkyle, ramifié ou non, ayant de 1 à 4 atomes de carbone, un radical alkoxy ayant de 1 à 10 atomes de carbone, un radical cycloaliphatique sustitué ou non, un radical thio-cycloaliphatique ou un radical de formule $-O-Si(CH_3)_2-R_8$, $R_8$ représentant un radical alkyle inférieur linéaire ou ramifié,
– $R_4$ et $R_5$, identiques ou différents, représentent un atome d'hydrogène, un radical alkyle inférieur, un radical hydroxyle ou un radical acyloxy inférieur, les sels desdits dérivés benzonaphtaléniques de formule (I).

2. Composés selon la revendication 1, caractérisés par le fait que le radical alkyle est pris dans le groupe constitué par un radical méthyle, éthyle, isopropyle, butyle et tertiobutyle.

3. Composés selon la revendication 1, caractérisés par le fait que le radical alkoxy est un radical ayant de 1 à 10 atomes de carbone et est pris dans le groupe constitué par un radical méthoxy, éthoxy, isopropoxy, hexyloxy et décyloxy.

4. Composés selon la revendication 1 caractérisés par le fait que le radical acyloxy inférieur est un radical ayant de 1 à 4 atomes de carbone et est pris dans le groupe constitué par un radical acétyloxy et propionyloxy.

5. Composés selon la revendication 1, caractérisés par le fait que le radical monohydroxyalkyle inférieur est un radical ayant 2 ou 3 atomes de carbone et est pris dans le groupe constitué par un radical hydroxy-2-éthyle et hydroxy-2-propyle.

6. Composés selon la revendication 1, caractérisés par le fait que le radical polyhydroxyalkyle est un radical ayant de 3 à 6 atomes de carbone et de 2 à 5 groupes hydroxyles et est pris dans le groupe constitué par un radical dihydroxy-2,3-propyle, dihydroxy-1,3-propyle, ou un reste du pentaérythritol.

7. Composés selon la revendication 1, caractérisés par le fait que le radical cycloaliphatique est un radical mono ou polycyclique et est pris dans le groupe constitué par le radical méthyl-1-cyclohexyle et le radical 1-adamantyle.

8. Composés selon la revendication 1, caractérisés par le fait que le radical thiocycloaliphatique est le radical 1-adamantylthio.

9. Composés selon la revendication 1, caractérisés par le fait que les radicaux r' et r", pris ensemble forment un hétérocycle pris dans le groupe consitué par un radical pipéridino, pipérazino, morpholino ou pyrrolidino.

10. Composés selon l'une quelconque des revendications 1 à 9 caractérisés par le fait qu'ils correspondent à la formule générale suivante:

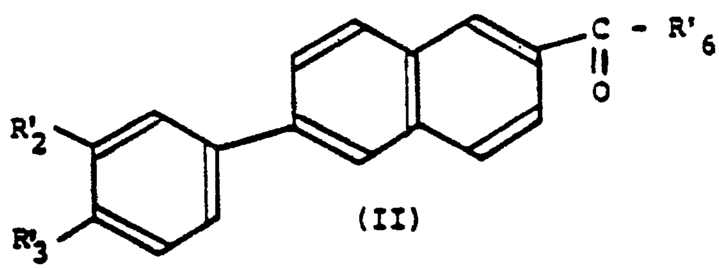

(II)

(II)

dans laquelle:

$R'_6$ représente un radical

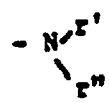

ou un radical $-OR'_7$

r' et r", identiques ou différents, représentent un atome d'hydrogène ou un radical alkyle inférieur ou pris ensemble forment un radical morpholino, $R'_7$ représentant un atome d'hydrogène ou un radical alkyle inférieur,

$R'_2$ représente un atome d'hydrogène, un radical alkyle, un radical alkoxy ou un radical 1-adamantyle,

et $R'_3$ représente un atome d'hydrogène, un radical hydroxyle, un radical alkyle, un radical alkoxy, ou un radical 1-adamantylthio.

11. Composés selon l'une quelconque des revendications précédentes caractérisés par le fait qu'ils sont pris dans le groupe constitué par

– l'acide 6-(3-méthylphényl)-2-naphtoïque et son ester méthylique,
– l'acide 6-(4-tertiobutyl phényl)-2-naphtoïque et son ester méthylique,
– l'acide 6-(3-tertiobutyl phényl)-2-naphtoïque et son ester méthylique,
– l'acide 6-(3,4-diméthoxy phényl)-2-naphtoïque et son ester méthylique,
– l'acide 6-[p-(1-adamantylthio)phényl]-2-naphtoïque et son ester méthylique,
– l'acide 6-[3-(1-adamantyl)-4-méthoxyphényl]-2-naphtoïque et son ester méthylique.
– L'Ester méthylique de l'acide 6-[3-(1-adamantyl-4-tert-butyldiméthylsilyloxyphenyl]-2-naphtoïque
– L'Ester méthylique de l'acide 6-[3-(1-adamantyl)-4-hydroxyphényl]-2-naphtoïque
– L'Acide 6-[ 3-(1-adamantyl)-4-hydroxyphényl]-2-naphtoïque
– L'Ester méthylique de l'acide 6-[3-(1-adamantyl)-4-bromo-1-décyloxyphényl]-2-naphtoïque
– L'Acide 6-[3-(1-adamantyl)-4-décyloxyphényl]-2-naphtoïque
– L'Ester méthylique de l'acide 6-[3-(1-adamantyl)-4-hexyloxyphényl]-2-naphtoïque
– L'Acide 6-[3-(1-adamantyl)-4-hexyloxyphényl-2-naphtoïque

– L'Ester méthylique de l'acide 6-[3-(1-adamantyl)-4-méthoxyphényl]-4-acétoxy-1-méthyl-2-naphtoïque
– L'Acide 6-[3-(1-adamantyl)-4-méthoxyphény]-4-hydroxy-1-méthyl-2-naphtoïque
– L'Ester méthylique de l'acide 6-[3-(1-adamantyl)-4-méthoxyphényl]-4-hydroxy-1-méthyl-2-naphtoïque
– L'Ester méthylique de l'acide 6-[3-(1-adamantyl)-4-méthoxyphényl]-1-méthyl-2-naphtoïque
– L'Acide 6-[3-(1-adamantyl)-4-méthoxyphényl]-1-méthyl-2-naphtoïque
– Le 6-[3-(1-adamantyl)-4-méthoxyphényl]-2-naphtalène méthanol
– L'Ethylamide de l'acide 6-[3-(1-adamantyl)-4-méthoxyphényl]-2-naphtoïque
– Le Morpholide de l'acide 6-[3-(1-adamantyl)-4-méthoxyphényl]-2-naphtoïque
– L'Ester méthylique de l'acide 6-(3-tert-butyl-4-méthoxyphényl)-2-naphtoïque
– L'Acide 6-(3-tert-butyl-4-méthoxyphényl)-2-naphtoïque
– L'Ester méthylique de l'acide 6-[3-(1,1-diméthyldécyl)-4-méthoxyphényl]-2-naphtoique, et
– L'Acide 6-[3-(1,1-diméthyldécyl)-4-méthoxyphényl)-2-naphtoïque

12. Procédé de préparation des composés selon l'une quelconque des revendications 1 à 11, caractérisé par le fait qu'il consiste à faire réagir par couplage, en milieu solvant anhydre et en présence, comme catalyseur de réaction, d'un métal de transition ou de l'un de ses complexes, le magnésien, le lithien ou le zincique d'un composé de la formule (III) suivante:

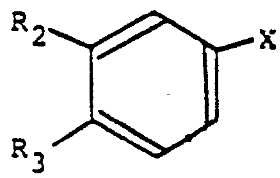

(III)

avec un composé halogéné du naphtalène, de formule (IV) suivante:

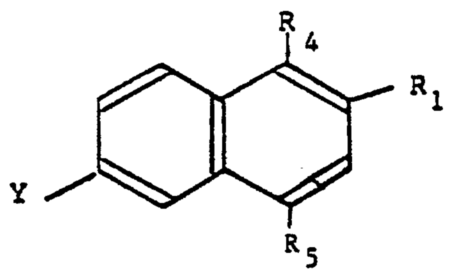

(IV)

dans lesquelles:
$R_1$ à $R_5$ ont les mêmes significations qu'à la revendication 1 et X et Y représentent Cl, Br, F ou I.

13. Procédé selon la revendication 12, caractérisé par le fait que la réaction de coupalge est effectuée entre –20 et +30°C.

14. Médicament caractérisé par le fait qu'il est un composé actif de formule (I) et/ou un de ses sels selon l'une quelconque des revendications 1 à 11.

15. Composition pharmaceutique caractérisée par le fait qu'elle contient dans un véhicule approprié pour une administration par voie entérale, parentérale, topique ou oculaire, au moins un composé actif de formule (I) et/ou un de ses sels selon l'une quelconque des revendications 1 à 11.

16. Composition selon la revendication 15 caractérisée par le fait qu'elle contient de 0,0005 à environ 5% en poids du composé actif.

17. Composition selon la revendication 15, caractérisée par le fait qu'elle est administrée à une dose journalière de 2 µg/kg à 2 mg/kg de poids corporel.

18. Utilisation du médicament selon la revendication 14 pour la préparation d'une composition pharmaceutique destinée au traitement des affections dermatologiques, rhumatismales, respiratoires ainsi qu'ophtalmologiques.

19 Composition cosmétique pour l'hygiène corporelle et capillaire, caractérisée par le fait qu'elle contient dans un véhicule cosmétique approprié au moins un composé actif de formule (I) selon l'une quelconque des revendications 1 à 11.

20. Composition cosmétique selon la revendication 19 caractérisée par le fait qu'elle contient le composé actif de formule (I) à une concentration comprise entre 0,0005 et 2%, et de préférence entre 0,01 et 1% en poids.

**Revendications pour l'Etat contractant: AT**

1. Procédé de préparation de composés benzonaphtaléniques répontant à la formule générale suivante:

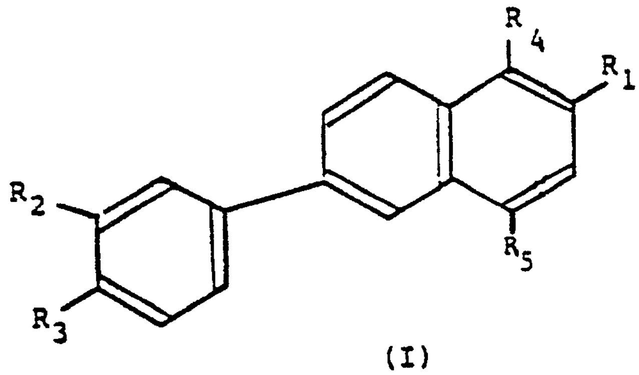

(I)

dans laquelle:
$R_1$ représente un radical

(i) $-\overset{\overset{O}{\|}}{C}-R_6$ ou (ii) $-CH_2OH$

$R_6$ représente un radical

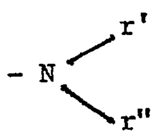

ou un radical $-OR_7$, $R_7$ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono- ou polyhydroxyalkyle, un radical aryle éventuellement sustitué ou un rest d'amino-acide ou de sucre aminé ou encore pris ensemble forment un hétérocycle,

$R_2$ représente un atome d'hydrogène, un radical alkyle ramifié ou non, ayant de 1 à 15 atomes de carbone, un radical alkoxy ayant de 1 à 4 atomes de carbone, ou un radical cycloaliphatique,

$R_3$ représente un atome d'hydrogène, un radical hydroxyle, un radical alkyle, ramifié ou non, ayant de 1 à 4 atomes de carbone, un radical alkoxy ayant de 1 à 10 atomes de carbone, un radical cycloaliphatique substitué ou non, un radical thio-cycloaliphatique ou un radical de formule $-O-Si(CH_3)_2-R_8$, $R_8$ représentant un radical alkyle inférieur linéaire ou ramifié,

$R_4$ et $R_5$, identiques ou différents, représentent un atome d'hydrogène, un radical alkyle inférieur, un radical hydroxyle ou un radical acyloxy inférieur, caractérisé par le fait qu'il consiste à faire réagir par couplage, en milieu solvant anhydre et en présence, comme catalyseur de réaction, d'un métal de transition ou de l'un de ses complexes, le magnésien, le lithien ou le zincique d'une composé de la folmule (III) suivante:

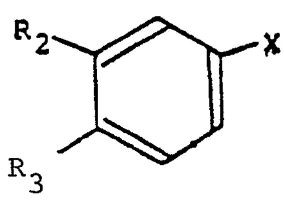

(III)

avec composé halogéné du naphtalène, de formule (IV) suivante:

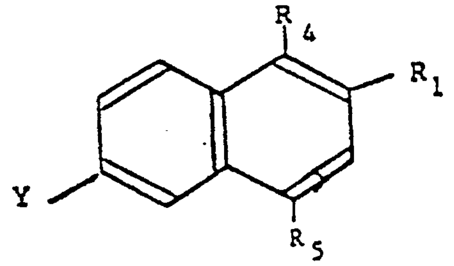

(IV)

dans lesquelles:
$R_1$ à $R_5$ ont les mêmes significations que ci-dessus, et X et Y représentent Cl, Br, F ou I.

2. Procédé selon la revendication 1, caractérisé par le fait que la réaction de couplage est effectuée entre −20 et +30°C.

3. Procédé selon la revendication 1 ou 2, caractérisé par le fait que les composés obtenus correspondent de préférence à la formule générale suivante:

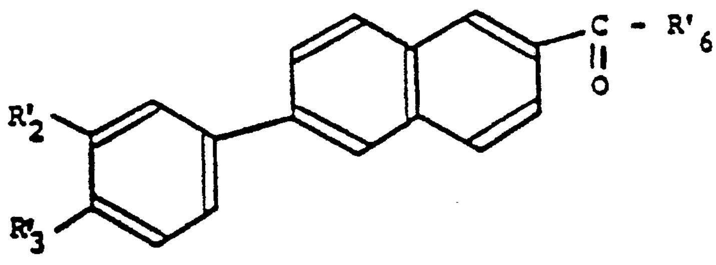

(II)

dans laquelle:
$R'_6$ représente un radical

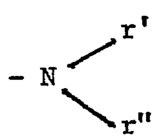

ou un radical $-OR'_7$

R' et R", identiques ou différents, représentent un atome d'hydrogène ou un radical alkyle inférieur ou pris ensemble forment un radical morpholine, $R'^7$ représentant un atome d'hydrogène ou un radical alkyle inférieur,

$R'_2$ représente un atome d'hydrogène, un radical alkyle, un radical alkoxy ou un radical 1-adamantyle, et

$R'_3$ représente un atome d'hydrogène, un radical hydroxyle, un radical alkyle, un radical alkoxy, ou un radical 1-adamantylthio.

4. Procédé selon l'une quelconque des revendications 1 à 3, caractérisé par le fait que les composés obtenus sont pris dans le groupe constitué par:

– l'acide 6-(3-méthylphényl)-2-naphtoïque et son ester méthylique,
– l'acide 6-(4-tertiobutyl phényl)-2-naphtoïque et son ester méthylique,
– l'acide 6-(3-tertiobutyl phényl)-2-naphtoïque et son ester méthylique,
– l'acide 6-(3,4-diméthoxy phényl)-2-naphtoïque et son ester méthylique,
– l'acide 6-[p-(1-adamantylthio)phényl]-2-naphtoïque et son ester méthylique,
– l'acide 6-[3-(1-adamantyl)-4-méthoxyphényl]-2-naphtoïque et son ester méthylique,
– l'ester méthylique de l'acide 6-[3-(1-adamantyl)-4-tert-butyldiméthylsilyloxyphényl]-2-naphtoïque,
– l'ester méthylique de l'acide 6-[3-(1-adamantyl)-4-hydroxyphényl]-2-naphtoïque,
– l'acide 6-[3-(1-adamantyl)-4-hydroxyphényl]-2-naphtoïque,
– l'ester méthylique de l'acide 6-[3-(1-adamantyl)-4-bromo-1-décyloxyphényl]-2-naphtoïque,
– l'acide 6-[3-(1-adamantyl)-4-décyloxyphényl]-2-naphtoïque,
– l'ester méthylique de l'acide 6-[3-(1-adamantyl)-4-hexyloxyphényl]-2-naphtoïque,
– l'acide 6-[3-(1-adamantyl)-4-hexyloxyphényl]-2-naphtoïque,
– l'ester méthylique de l'acide 6-[3-(1-adamantyl)-4-méthoxyphényl]-4-acétoxy-1-méthyl-2-naphtoïque,
– l'acide 6-[3-(1-adamantyl)-4-méthoxyphényl]-4-hydroxy-1-méthyl-2-naphtoïque,

– l'ester méthylique de l'acide 6-[3-(1-adamantyl)-4-méthoxyphényl]-4-hydroxy-1-méthyl-2-naphtoïque,
– l'ester méthylique de l'acide 6-[3-(1-adamantyl)-4-méthoxyphényl]-1-méthyl-2-naphtoïque,
– l'acide 6-[3-(1-adamantyl)-4-méthoxyphényl]-1-méthyl-2-naphtoïque,
– le 6-[3-(1-adamantyl)-4-méthoxyphényl]-2-naphtalène méthanol,
– l'éthylamide de l'acide 6-[3-(1-adamantyl)-4-méthoxyphényl]-2-naphtoïque,
– le morpholide de l'acide 6-[3-(1-adamantyl)-4-méthoxyphényl]-2-naphtoïque,
– l'ester méthylique de l'acide 6-(3-tert-butyl-4-méthoxyphényl)-2-naphtoïque,
– l'acide 6-(3-tert-butyl-4-méthoxyphényl)-2-naphtoïque,
– l'ester méthylique de l'acide 6-[3-(1,1-diméthyldécyl)-4-méthoxyphényl]-2-naphtoïque, et
– l'acide 6-[3-(1,1-diméthyldécyl)-4-méthoxyphényl]-2-naphtoïque.

5. Composition cosmétique pour l'hygiène corporelle et capillaire, caractérisée par le fait qu'elle contient dans un véhicule cosmétique approprié au moins un composé actif de formule générale suivante:

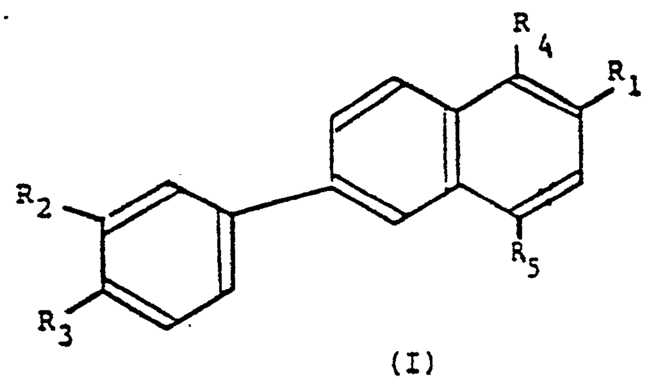

(I)

dans laquelle:
$R_1$ représente:

$$(i)\ -\underset{\underset{O}{\|}}{C}-R_6 \quad \text{ou} \quad (ii)\ -CH_2OH$$

$R_6$ représente un radical

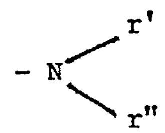

ou un radical $-OR_7$, $R_7$ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono- ou polyhydroxyalkyle, un radical aryle éventuellement substitué ou un reste d'amino-acide ou de sucre aminé ou encore pris ensemble forment un hétérocycle,

$R_2$ représente un atome d'hydrogène, un radical alkyle, ramifié ou non, ayant de 1 à 15 atomes de carbone, un radical alkoxy ayant de 1 à 4 atomes de carbone, ou un radical cycloaliphatique,

$R_3$ représente un atome d'hydrogène, un radical hydroxyle, un radical alkyle, ramifié ou non, ayant de 1 à 4 atomes de carbone, un radical alkoxy ayant de 1 à 10 atomes de carbone, un radical cycloaliphatique substitué ou non, un radical thio-cycloaliphatique ou un radical de formule $-O-Si(CH_3)_2-R_8$, $R_8$ représentant un radical alkyle inférieur linéaire ou ramifié,

$R_4$ et $R_5$, identiques ou différents, représentent un atome d'hydrogène, un radical alkyle inférieur, un radical hydroxyle ou un radical acyloxy inférieur, les sels desdits dérivés benzonaphtaléniques de formule (I).

6. Composition cosmétique selon la revendication 5, caractérisée par le fait qu'elle contient le composé actif de formule (I) à une concentration comprise entre 0,0005 et 2% et de préférence entre 0,01 et 1% en poids.

**Patentansprüche für die Vertragsstaaten BE, CH, DE, FR, GB, IT, LI, LU, NL, SE**

1. Benzonaphthalin-Verbindungen der folgenden allgemeinen Formel

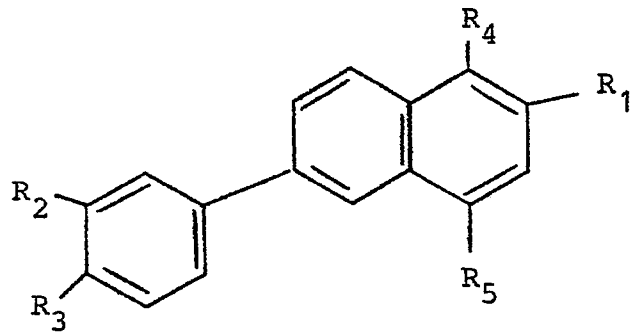

(I)

worin
$R_1$ für

(i) 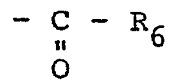

oder

(ii) $-CH_2OH$ steht,

steht, wobei $R_6$ für einen Rest

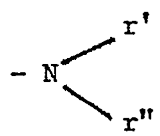

oder einen Rest $-OR_7$ steht, wobei $R_7$ ein Wasserstoffatom, einen Alkylrest mit 1 bis 20 Kohlenstoff-atomen, einen Monohydroxyalkylrest oder einen Polyhydroxyalkylrest bedeutet,
r' und r'' ein Wasserstoffatom, einen niedrigen Alkylrest, einen Mono- oder Polyhydroxyalkylrest, einen gegebenenfalls substituierten Arylrest oder einen Rest einer Aminosäure oder eines aminierten Zuckers bedeuten oder zusammen auch einen Heterocyclus bilden,

$R_2$ ein Wasserstoffatom, einen gegebenenfalls verzweigten Alkylrest mit 1 bis 15 Kohlenstoffatomen, einen Alkoxyrest mit 1 bis 4 Kohlenstoffatomen oder einen cycloaliphatischen Rest bedeutet,

$R_3$ ein Wasserstoffatom, einen Hydroxyrest, einen gegebenenfalls verzweigten Alkylrest mit 1 bis 4 Kohlenstoffatomen, einen Alkoxyrest mit 1 bis 10 Kohlenstoffatomen, einen gegebenenfalls substituierten cycloaliphatischen Rest, einen thiocycloaliphatischen Rest oder einen Rest der Formel $-O-(CH_3)_2-R_8$ bedeutet, wobei $R_8$ für einen linearen oder verzweigten niedrigen Alkylrest steht, und

$R_4$ und $R_5$, die gleich oder verschieden sind, ein Wasserstoffatom, einen niedrigen Alkylrest, einen Hydroxyrest oder einen niedrigen Acyloxyrest bedeuten, sowie die Salze der Benzonaphthalin-Derivate der Formel (I).

2. Verbindungen nach Anspruch 1, dadurch gekennzeichnet, daß es sich bei dem Alkylrest um einen Methyl-, Ethyl-, Isopropyl-, Butyl- oder tert.-Butylrest handelt.

3. Verbindungen nach Anspruch 1, dadurch gekennzeichnet, daß der Alkoxyrest ein Rest mit 1 bis 10 Kohlenstoffatomen ist und einen Methoxy-, Ethoxy-, Isopropoxy-, Hexyloxy- oder Decyloxyrest darstellt.

4. Verbindungen nach Anspruch 1, dadurch gekennzeichnet, daß der niedrige Acyloxyrest ein Rest mit 1 bis 4 Kohlenstoffatomen ist und einen Acetyloxy- oder Propionyloxyrest darstellt.

5. Verbindungen nach Anspruch 1, dadurch gekennzeichnet, daß der niedrige Monohydroxyalkylrest ein Rest mit 2 oder 3 Kohlenstoffatomen ist und einen 2-Hydroxyethyl- oder 2-Hydroxypropylrest darstellt.

6. Verbindungen nach Anspruch 1, dadurch gekennzeichnet, daß der Polyhydroxyalkylrest eine Rest mit 3 bis 6 Kohlenstoffatomen und mit 2 bis 5 Hydroxygruppen ist und einen 2,3-Dihydroxypropylrest, einen 1,3-Dihydroxypropylrest oder einen Pentaerythritrest darstellt.

7. Verbindungen nach Anspruch 1, dadurch gekennzeichnet, daß der cycloaliphatische Rest ein mono- oder polycyclischer Rest ist und den 1-Methylcyclohexylrest oder 1-Adamantylrest darstellt.

8. Verbindungen nach Anspruch 1, dadurch gekennzeichnet, daß der thiocycloaliphatische Rest der 1-Adamantylthiorest ist.

9. Verbindungen nach Anspruch 1, dadurch gekennzeichnet, daß die Reste r' und r" zusammen einen Heterocyclus bilden und für einen Piperidino-, Piperazino-, Morpholino- oder Pyrrolidinorest stehen.

10. Verbindungen nach einem der Ansprüche 1 bis 9 der folgenden allgemeinen Formel

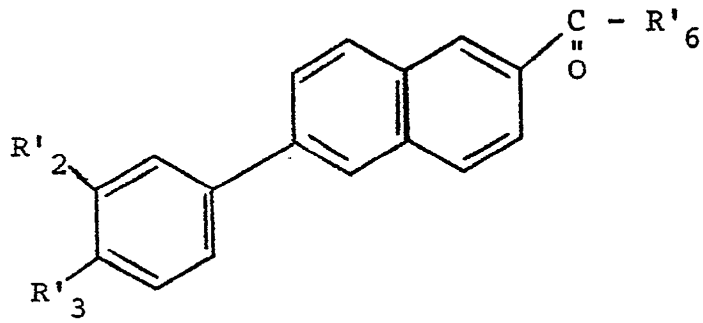

(II)

worin
R'$_6$ für einen Rest

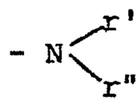

oder einen Rest –OR'$_7$ steht, wobei r' und r", die gleich oder verschieden sind, ein Wasserstoffatom oder einen niedrigen Alkylrest bedeuten oder zusammen einen Morpholinorest bilden und R'$_7$ ein Wasserstoffatom oder einen niedrigen Alkylrest bedeutet,

R'$_2$ ein Wasserstoffatom oder einen Alkylrest, einen Alkoxyrest oder den 1-Adamantylrest bedeutet und

R'$_3$ ein Wasserstoffatom, einen Hydroxyrest, einen Alkylrest, einen Alkoxyrest oder den 1-Adamantylthiorest bedeutet.

11. Verbindungen nach einem der vorhergehenden Ansprüche, nämlich:
6-(3-Methylphenyl)-2-naphthoesäure und deren Methylester,
6-(4-tert.-Butylphenyl)-2-naphthoesäure und deren Methylester,
6-(3-tert.-Butylphenyl)-2-naphthoesäure und deren Methylester,
6-(3,4-Dimethoxyphenyl)-2-naphthoesäure und deren Methylester,
6-[p-(1-Adamantylthio)phenyl]-2-naphthoesäure und deren Methylester,
6-[3-(1-Adamantyl)-4-methoxyphenyl]-2-naphthoesäure und deren Methylester,
6-[3-(1-Adamantyl)-4-tert.-butyldimethylsilyloxyphenyl]-2-naphthoesäure-methylester,
6-[3-(1-Adamantyl)-4-hydroxyphenyl]-2-naphthoesäure-methylester,
6-[3-(1-Adamantyl)-4-hydroxyphenyl]-2-naphthoesäure,
6-[3-(1-Adamantyl)-4-brom-1-decyloxyphenyl]-2-naphthoesäure-methylester,
6-[3-(1-Adamantyl)-4-decyloxyphenyl]-2-naphthoesäure,
6-[3-(1-Adamantyl)-4-hexyloxyphenyl]-2-naphthoesäure-methylester,
6-[3-(1-Adamantyl)-4-hexyloxyphenyl]-2-naphthoesäure,
6-[3-(1-Adamantyl)-4-methoxyphenyl]-4-acetoxy-1-methyl-2-naphthoesäure-methylester,
6-[3-(1-Adamantyl)-4-methoxyphenyl]-4-hydroxy-1-methyl-2-naphthoesäure,
6-[3-(1-Adamantyl)-4-methoxyphenyl]-4-hydroxy-1-methyl-2-naphthoesäure-methylester,
6-[3-(1-Adamantyl)-4-methoxyphenyl]-1-methyl-2-naphthoesäure-methylester,
6-[3-(1-Adamantyl)-4-methoxyphenyl]-1-methyl-2-naphthoesäure,
6-[3-(1-Adamantyl)-4-methoxyphenyl]-2-naphthalinmethanol,
6-[3-(1-Adamantyl)-4-methoxyphenyl]-2-naphthoesäure-ethylamid,
6-[3-(1-Adamantyl)-4-methoxyphenyl]-2-naphthoesäure-morpholid,
6-(3-tert.-Butyl-4-methoxyphenyl)-2-naphthoesäure-methylester,
6-(3-tert.-Butyl-4-methoxyphenyl)-2-naphthoesäure,
6-[3-(1,1-Dimethyldecyl)-4-methoxyphenyl]-2-naphthoesäure-methylester, und
6-[3-(1,1-Dimethyldecyl)-4-methoxyphenyl]-2-naphthoesäure.

12. Verfahren zur Herstellung der Verbindungen nach einem der Ansprüche 1 bis 11, dadurch gekennzeichnet, daß man in einem wasserfreien Lösungsmittelmilieu und in Anwesenheit eines Übergangsmetalls oder eines seiner Komplexe als Reaktionskatalysator das Magnesium-, Lithium- oder Zinkderivat einer Verbindung der folgenden Formel (III):

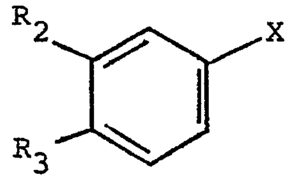

(III)

mit einer halogenierten Naphthalinverbindung der folgenden Formel (IV):

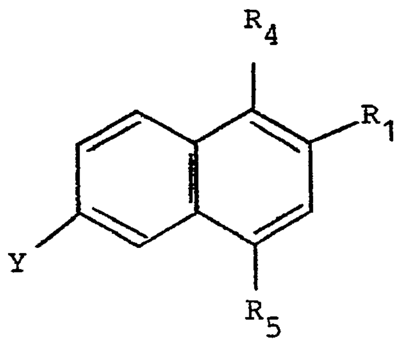

(IV)

worin
$R_1$ bis $R_5$ die in Anspruch 1 angegebenen Bedeutungen besitzen und
X und Y für Cl, Br, F oder I stehen,
umsetzt.

13. Verfahren nach Anspruch 12, dadurch gekennzeichnet, daß man die Umsetzung bei –20 bis +30°C durchführt.

14. Medikament, dadurch gekennzeichnet, daß es ein Wirkstoff der Formel (I) und/oder eine Salz davon nach einem der Ansprüche 1 bis 11 ist.

15. Pharmazeutisches Mittel, dadurch gekennzeichnet, daß es in einem für eine enterale, parenterale, topische oder okulare Verabreichung geeigneten Träger mindestens einen Wirkstoff der Formel (I) und/oder ein Salz davon nach einem der Ansprüche 1 bis 11 enthält.

16. Mittel nach Anspruch 15, dadurch gekennzeichnet, daß es 0,0005 bis etwa 5 Gew.-% des Wirkstoffs enthält.

17. Mittel nach Anspruch 15, dadurch gekennzeichnet, daß es in einer täglichen Dosis von 2 µg/kg bis 2 mg/kg Körpergewicht verabreicht wird.

18. Verwendung des Medikaments nach Anspruch 14 zur Herstellung eines pharmazeutischen Mittels zur Behandlung von dermatologischen, rheumatischen, respiratorischen sowie ophthalmologischen Störungen.

19. Kosmetisches Mittel zur Haar- und Körperhygiene, dadurch gekennzeichnet, daß es in einem geeigneten kosmetischen Träger mindestens einen Wirkstoff der Formel (I) nach einem der Ansprüche 1 bis 11 enthält.

20. Kosmetisches Mittel nach Anspruch 19, dadurch gekennzeichnet, daß es den Wirkstoff der Formel (I) in einer Konzentration von 0,0005 bis 2% und vorzugsweise von 0,01 bis 1 Gew.-% enthält.

**Claims for the Contracting States BE, CH, DE, FR, GB, IT, LI, LU, NL, SE**

1. Benzonaphthalene compounds characterized in that they correspond to the following general formula:

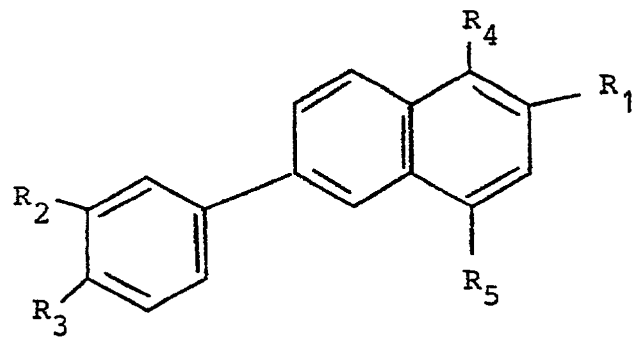

(I)

in which
$R_1$ denotes:

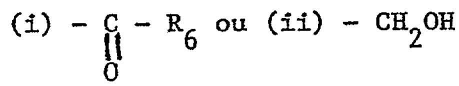

$R_6$ denotes a radical

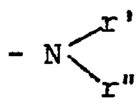

or a radical $-OR_7$, $R_7$ denoting a hydrogen atom, an alkyl radical having from 1 to 20 carbon atoms, a monohydroxyalkyl radical or a polyhydroxyalkyl radical, r' and r" denoting a hydrogen atom, a lower alkyl radical, a mono- or polyhydroxyalkyl radical, an optionally substituted aryl radical or an amino acid residue or amino sugar residue or alternatively taken together form a heterocycle,

$R_2$ denotes a hydrogen atom, an alkyl radical, branched or otherwise, having from 1 to 15 carbon atoms, an alkoxy radical having from 1 to 4 carbon atoms or a cycloaliphatic radical,

$R_3$ denotes a hydrogen atom, a hydroxyl radical, an alkyl radical, branched or otherwise, having from 1 to 4 carbon atoms, an alkoxy radical having from 1 to 10 carbon atoms, a substituted or unsubstituted cycloaliphatic radical, a thiocycloaliphatic radical or a radical of formula $-O-Si(CH_3)_2-R_8$, $R_8$ denoting a linear or branched lower alkyl radical, and

$R_4$ and $R_5$, which may be identical or different, denote a hydrogen atom, a lower alkyl radical, a hydroxyl radical or a lower acyloxy radical,
and the salts of the said benzonaphthalene derivatives of formula (I).

2. Compounds according to Claim 1, characterized in that the alkyl radical is selected from the group consisting of a methyl, ethyl, isopropyl, butyl and tert-butyl radical.

3. Compounds according to Claim 1, characterized in that the alkoxy radical is a radical having from 1 to 10 carbon atoms and is selected from the group consisting of a methoxy, ethoxy, isopropoxy, nexyloxy and decyloxy radical.

4. Compounds according to Claim 1, characterized in that the lower acyloxy radical is a radical having from 1 to 4 carbon atoms and is selected from the group consisting of an acetyloxy and propionyloxy radical.

5. Compounds according to Claim 1, characterized in that the lower monohydroxy alkyl radical is a radical having 2 or 3 carbon atoms and is selected from the group consisting of a 2-hydroxyethyl and 2-hydroxypropyl radical.

6. Compounds according to Claim 1, characterized in that the polyhydroxyalkyl radical is a radical having from 3 to 6 carbon atoms and from 2 to 5 hydroxyl groups, and is selected from the group consisting of a 2,3-dihydroxypropyl and 1,3-dihydroxypropyl radical or a pentaerythritol residue.

7. Compounds according to Claim 1, characterized in that the cycloaliphatic radical is a mono- or polycyclic radical and is selected from the group consisting of a 1-methylcyclohexyl radical and a 1-adamantyl radical.

8. Compounds according to Claim 1, characterized in that the thiocycloaliphatic radical is a 1-adamantylthio radical.

9. Compounds according to Claim 1, characterized in that the radicals r' and r", taken together, form a heterocycle selected from the group consisting of a piperidinyl, piperazino, morpholino or pyrrolidino radical.

10. Compounds according to any one of Claims 1 to 9, characterized in that they correspend to the following general formula:

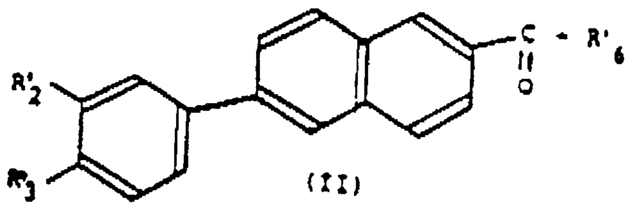

(II)

(II)

in which:
R'$_6$ denotes a radical

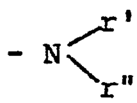

or a radical –OR'$_7$

r' and r", which may be identical or different, denote a hydrogen atom or a lower alkyl radical, R'$_7$ denoting a hydrogen atom or a lower alkyl radical,

R'$_2$ denotes a hydrogen atom, an alkyl radical, an alkoxy radical or a 1-adamantyl radical, and

R'$_3$ denotes a hydrogen atom, a hydroxyl radical, an alkyl radical, an alkoxy radical or a 1-adamantylthio radical.

11. Compounds according to any one of the preceding claims, characterized in that they are selected from the group consisting of

– 6-(3-methylphenyl)-2-naphthoic acid and its methyl ester,
– 6-(4-tert-butylphenyl)-2-naphthoic acid and its methyl ester,
– 6-(3-tert-butylphenyl)-2-naphthoic acid and its methyl ester,
– 6-(3,4-dimethoxyphenyl)-2-naphthoic acid and its methyl ester,
– 6-[p-(1-adamantylthio)phenyl]-2-naphthoic acid and its methyl ester,
– 6-[3-(1-adamantyl)-4-methoxyphenyl]-2-naphthoic acid and its methyl ester,
– 6-[3-(1-adamantyl)-4-tert-butyldimethylsilyloxyphenyl]-2-naphthoic acid methyl ester,
– 6-[3-(1-adamantyl)-4-hydroxyphenyl]-2-naphthoic acid methyl ester,
– 6-[3-(1-adamantyl)-4-hydroxyphenyl]-2-naphthoic acid,
– 6-[3-(1-adamantyl)-4-bromo-1-decyloxyphenyl]-2-naphthoic acid methyl ester,
– 6-[3-(1-adamantyl)-4-decyloxyphenyl]-2-naphthoic acid,
– 6-[3-(1-adamantyl)-4-hexyloxyphenyl]-2-naphthoic acid methyl ester,
– 6-[3-(1-adamantyl)-4-hexyloxyphenyl]-2-naphthoic acid,
– 6-[3-(1-adamantyl)-4-methoxyphenyl]-4-acetoxy-1-methyl-2-naphthoic acid methyl ester,
– 6-[3-(1-adamantyl)-4-methoxyphenyl]-4-hydroxy-1-methyl-2-naphthoic acid,
– 6-[3-(1-adamantyl)-4-methoxyphenyl]-4-hydroxy-1-methyl-2-naphthoic acid methyl ester,
– 6-[3-(1-adamantyl)-4-methoxyphenyl]-1-methyl-2-naphthoic acid methyl ester,
– 6-[3-(1-adamantyl)-4-methoxyphenyl]-1-methyl-2-naphthoic acid,
– 6-[3-(1-adamantyl)-4-methoxyphenyl]-2-naphthalenemethanol,
– 6-[3-(1-adamantyl)-4-methoxyphenyl]-2-naphthoic acid ethylamide,
– 6-[3-(1-adamantyl)-4-methoxyphenyl]-2-naphthoic acid morpholide,
– 6-(3-tert-butyl-4-methoxyphenyl)-2-naphthoic acid methyl ester,
– 6-(3-tert-butyl-4-methoxyphenyl)-2-naphthoic acid,
– 6-[3-(1,1-dimethyldecyl)-4-methoxyphenyl]-2-naphthoic acid methyl ester, and
– 6-[3-(1,1-dimethyldecyl)-4-methoxyphenyl]-2-naphthoic acid.

12. Process for preparing the compounds according to any one of Claims 1 to 11, characterized in that it consists in reacting, by coupling, in an anhydrous solvent medium and in the presence, as a reaction catalyst, of a transition metal or one of its complexes, the magnesium, lithium or zinc derivative of a compound of the following formula (III):

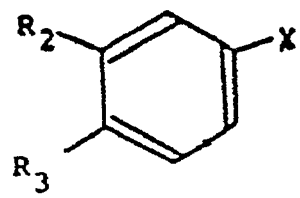

(III)

with a halogenated naphthalene compound of the following formula (IV):

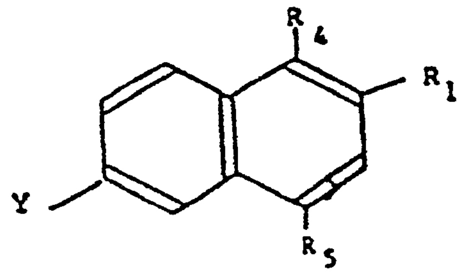

(IV)

in which:
$R_1$ to $R_5$ have the same meanings as in Claim 1 and X and Y denote Cl, Br, F or I.

13. Process according to Claim 12, characterized in that the coupling reaction is performed at between −20 and +30°C.

14. Medicinal product, characterized in that it is an active compound of formula (I) and/or one of its salts according to any one of Claims 1 to 11.

15. Pharmaceutical composition, characterized in that it contains at least one active compound of formula (I) and/or one of its salts according to any one of Claims 1 to 11, in a vehicle suitable for enteral, parenteral, topical or ocular administration.

16. Composition according to Claim 15, characterized in that it contains from 0.0005 to approximately 5% by weight of reactive compound.

17. Composition according to Claim 15, characterized in that it is administered at a daily dose of 2 µg/kg to 2 mg/kg of body weight.

18. Use of the medicinal product according to Claim 14, for the preparation of a pharmaceutical composition intended for the treatment of dermatological, rheumatic, respiratory and also ophthalmological conditions.

19. Cosmetic composition for body and hair hygiene, characterized in that it contains at least one active compound of formula (I) according tc any one of Claims 1 to 11, in a suitable cosmetic vehicle.

20. Cosmetic composition according to Claim 19, characterized in that it contains the active compound of formula (I) at a concentration of between 0.0005 and 2%, preferably between 0.01 and 1%, by weight.

**Patentansprüche für den Vertragsstaat AT**

1. Verfahren zur Herstellung der Benzonaphthalin-Verbindungen der folgenden allgemeinen Formel:

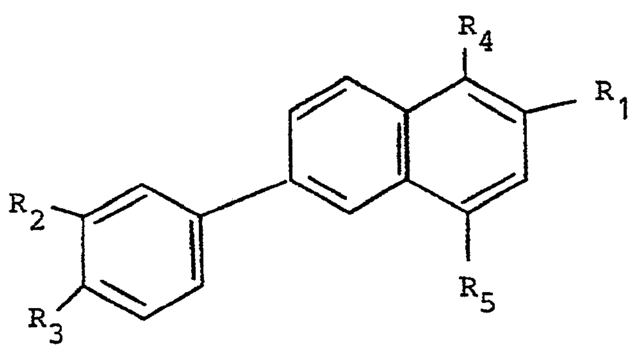

(I)

worin
$R_1$ für

(i) 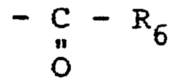

oder

(ii) $-CH_2OH$ steht,

wobei $R_6$ für einen Rest

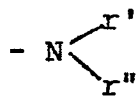

oder einen Rest $-OR_7$ steht, wobei

$R_7$ ein Wasserstoffatom, einen Alkylrest mit 1 bis 20 Kohlenstoffatomen, einen Monohydroxyalkylrest oder einen Polyhydroxyalkylrest bedeutet,

r' und r" ein Wasserstoffatom, einen niedrigen Alkylrest, einen Mono- oder Polyhydroxyalkylrest, einen gegebenenfalls substituierten Arylrest oder einen Rest einer Aminosäure oder eines aminierten Zuckers bedeuten oder zusammen auch einen Heterocyclus bilden,

$R_2$ ein Wasserstoffatom, einen gegebenenfalls verzweigten Alkylrest mit 1 bis 15 Kohlenstoffatomen, einen Alkoxyrest mit 1 bis 4 Kohlenstoffatomen oder einen cycloaliphatischen Rest bedeutet,

$R_3$ ein Wasserstoffatom, einen Hydroxyrest, einen gegebenenfalls verzweigten Alkylrest mit 1 bis 4 Kohlenstoffatomen, einen Alkoxyrest mit 1 bis 10 Kohlenstoffatomen, einen gegebenenfalls substituierten cycloaliphatischen Rest, einen thiocycloaliphatischen Rest oder einen Rest der Formel $-O-Si-(CH_3)_2-R_8$ bedeutet, wobei $R_8$ für einen linearen oder verzweigten niedrigen Alkylrest steht, und

$R_4$ und $R_5$, die gleich oder verschieden sind, ein Wasserstoffatom, einen niedrigen Alkylrest, einen Hydroxyrest oder einen niedrigen Acyloxyrest bedeuten, dadurch gekennzeichnet, daß man in einem wasserfreien Lösungsmittelmilieu und in Anwesenheit eines Übergangsmetalls oder eines seiner Komplexe als Reaktionskatalysator das Magenesium-, Lithium- oder Zinkderivat einer Verbindung der folgenden Formel (III):

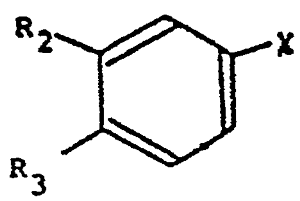

(III)

mit einer halogenierten Naphthalinverbindung der folgenden Formel (IV):

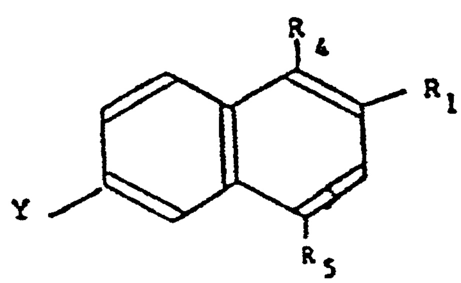

(IV)

worin

$R_1$ bis $R_5$ die in Anspruch 1 angegebenen Bedeutungen besitzen und

X und Y für Cl, Br, T oder I stehen,

umsetzt.

2. Verfahren nach Anspruch 1, dadurch gekennzeichnet, daß man die Umsetzung bei −20 bis +30°C durchführt.

3. Verfahren nach Anspruch 1 oder 2, dadurch gekennzeichnet, daß die erhaltenen Verbindungen vorzugsweise der folgenden allgemeinen Formel (II) entsprechen:

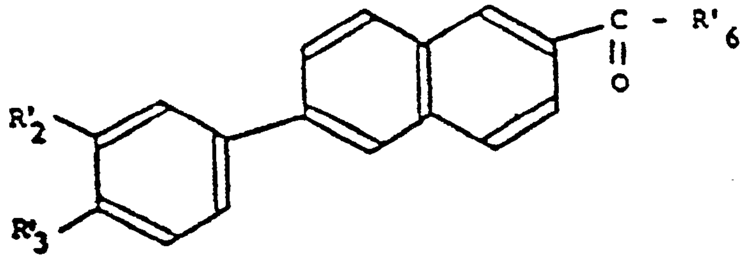

(II)

worin
R'$_6$ für einen Rest

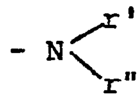

oder einen Rest –OR'$_7$ steht, wobei
r' und r'', die gleich oder verschieden sind, ein Wasserstoffatom oder einen niedrigen Alkylrest bedeuten oder zusammen einen Morpholinorest bilden und
R'$_7$ ein Wasserstoffatom oder einen niedrigen Alkylrest bedeutet,
R'$_2$ ein Wasserstoffatom oder einen Alkylrest, einen Alkoxyrest oder den 1-Adamantylrest bedeutet und
R'$_3$ ein Wasserstoffatom, einen Hydroxyrest, einen Alkylrest, einen Alkoxyrest oder den 1-Adamantylthiorest bedeutet.

4. Verfahren nach einem der Ansprüche 1 bis 3, dadurch gekennzeichnet, daß die erhaltenen Verbindungen ausgewählt sind unter:
6-(3-Methylphenyl)-2-naphthoesäure und deren Methylester,
6-(4-tert.-Butylphenyl)-2-naphthoesäure und deren Methylester,
6-(3-tert.-Butylphenyl)-2-naphthoesäure und deren Methylester,
6-(3,4-Dimethoxyphenyl)-2-naphthoesäure und deren Methylester,
6-[p-(1-Adamantylthio)phenyl]-2-naphthoesäure und deren Methylester,
6-[3-(1-Adamantyl)-4-methoxyphenyl]-2-naphthoesäure und deren Methylester,
6-[3-(1-Adamantyl)-4-tert.-butyldimethylsilyloxyphenyl]-2-naphthoesäure-methylester,
6-[3-(1-Adamantyl)-4-hydroxyphenyl]-2-naphthoesäure-methylester,
6-[3-(1-Adamantyl)-4-hydroxyphenyl]-2-naphthoesäure,
6-[3-(1-Adamantyl)-4-brom-1-decyloxyphenyl]-2-naphthoesäure-methylester,
6-[3-(1-Adamantyl)-4-decyloxyphenyl]-2-naphthoesäure,
6-[3-(1-Adamantyl)-4-hexyloxyphenyl]-2-naphthoesäure-methylester,
6-[3-(1-Adamantyl)-4-hexyloxyphenyl]-2-naphthoesäure,
6-[3-(1-Adamantyl)-4-methoxyphenyl]-4-acetoxy-1-methyl-2-naphthoesäure-methylester,
6-[3-(1-Adamantyl)-4-methoxyphenyl]-4-hydroxy-1-methyl-2-naphthoesäure,
6-[3-(1-Adamantyl)-4-methoxyphenyl]-4-hydroxy-1-methyl-2-naphthoesäure-methylester,
6-[3-(1-Adamantyl)-4-methoxyphenyl]-1-methyl-2-naphthoesäure-methylester,
6-[3-(1-Adamantyl)-4-methoxyphenyl]-1-methyl-2-naphthoesäure,
6-[3-(1-Adamantyl)-4-methoxyphenyl]-2-naphthalinmethanol,
6-[3-(1-Adamantyl)-4-methoxyphenyl]-2-naphthoesäure-ethylamid,
6-[3-(1-Adamantyl)-4-methoxyphenyl]-2-naphthoesäure-morpholid,
6-(3-tert.-Butyl-4-methoxyphenyl)-2-naphthoesäure-methylester,
6-(3-tert.-Butyl-4-methoxyphenyl)-2-naphthoesäure,
6-[3-(1,1-Dimethyldecyl)-4-methoxyphenyl]-2-naphthoesäure-methylester, und
6-[3-(1,1-Dimethyldecyl)-4-methoxyphenyl]-2-naphthoesäure.

5. Kosmetisches Mittel zur Haar- und Körperhygiene, dadurch gekennzeichnet, daß es in einem geeigneten kosmetischen Träger mindestens einen Wirkstoff der folgenden allgemeinen Formel enthält:

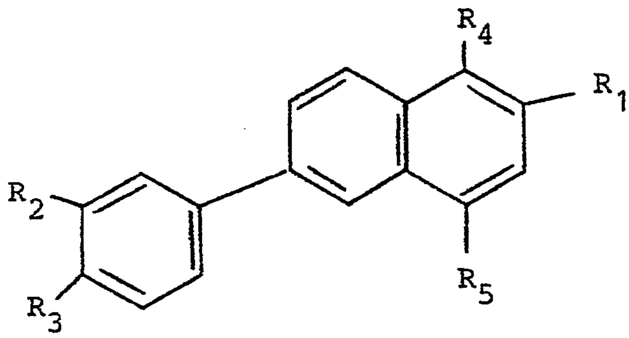

(I)

worin
$R_1$ für

(i) 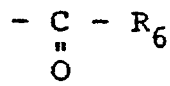

oder

(ii) $-CH_2OH$ steht,

wobei $R_6$ für einen Rest

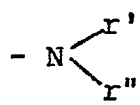

oder einen Rest $-OR_7$ steht, wobei
$R_7$ ein Wasserstoffatom, einen Alkylrest mit 1 bis 20 Kohlenstoffatomen, einen Monohydroxyalkylrest oder einen Polyhydroxyalkylrest bedeutet,
r' und r" ein Wasserstoffatom, einen niedrigen Alkylrest, einen Mono- oder Polyhydroxyalkylrest, einen gegebenenfalls substituierten Arylrest oder einen Rest einer Aminosäure oder eines aminierten Zuckers bedeuten oder zusammen auch einen Heterocyclus bilden,

$R_2$ ein Wasserstoffatom, einen gegebenenfalls verzweigten Alkylrest mit 1 bis 15 Kohlenstoffatomen, einen Alkoxyrest mit 1 bis 4 Kohlenstoffatomen oder einen cycloaliphatischen Rest bedeutet,

$R_3$ ein Wasserstoffatom, einen Hydroxyrest, einen gegebenenfalls verzweigten Alkylrest mit 1 bis 4 Kohlenstoffatomen, einen Alkoxyrest mit 1 bis 10 Kohlenstoffatomen, einen gegebenenfalls substituierten cycloaliphatischen Rest, einen thiocycloaliphatischen Rest oder einen Rest der Formel $-O-Si-(CH_3)_2-R_8$ bedeutet, wobei $R_8$ für einen linearen oder verzweigten niedrigen Alkylrest steht, und

$R_4$ und $R_5$, die gleich oder verschieden sind, ein Wasserstoffatom, einen niedrigen Alkylrest, einen Hydroxyrest oder einen niedrigen Acyloxyrest bedeuten, sowie die Salze der Benzonaphthalin-Derivate der Formel (I).

6. Kosmetisches Mittel nach Anspruch 5, dadurch gekennzeichnet, daß es den Wirkstoff der Formel (I) in einer Konzentration von 0,0005 bis 2% und vorzugsweise von 0,01 bis 1 Gew.-% enthält.

## Claims for the contracting state AT

1. Process for preparing benzonaphthalene compounds corresponding to the following general formula:

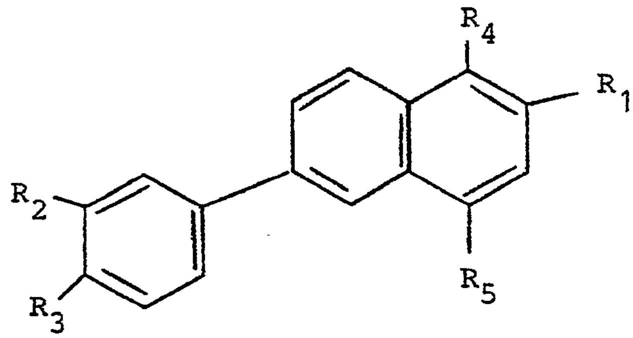

(I)

in which
$R_1$ denotes:

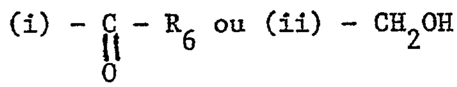

$R_6$ denotes a radical

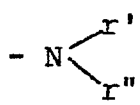

or a radical $-OR_7$, $R_7$ denoting a hydrogen atom, an alkyl radical having from 1 to 20 carbon atoms, a monohydroxyalkyl radical or a polyhydroxyalkyl radical, r' and r" denoting a hydrogen atom, a lower alkyl radical, a mono- or polyhydroxyalkyl radical, an optionally substituted aryl radical or an amino acid residue or amino sugar residue or alternatively taken together form a heterocycle,

$R_2$ denotes a hydrogen atom, an alkyl radical, branched or otherwise, having from 1 to 15 carbon atoms, an alkoxy radical having from 1 to 4 carbon atoms or a cycloaliphatic radical,

$R_3$ denotes a hydrogen atom, a hydroxyl radical, an alkyl radical, branched or otherwise, having from 1 to 4 carbon atoms, an alkoxy radical having from 1 to 10 carbon atoms, a substituted or unsubstituted cycloaliphatic radical, a thiocycloaliphatic radical or a radical of formula $-O-Si(CH_3)_2-R_8$, $R_8$ denoting a linear or branched lower alkyl radical, and

$R_4$ and $R_5$, which may be identical or different, denote a hydrogen atom, a lower alkyl radical, a hydroxyl radical or a lower acyloxy radical, characterized in that it consists in reacting, by coupling, in an anhydrous solvent medium and in the presence, as a reaction catalyst, of a transition metal or one of its complexes, the magnesium, lithium, or zinc derivative of a compound of the following formula (III):

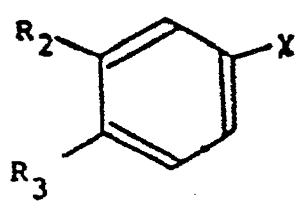

(III)

with a halogenated naphthalene compound of the following formula (IV):

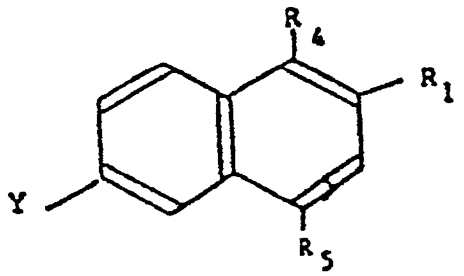

(IV)

in which:
$R_1$ to $R_5$ have the same meanings as above, and X and Y denote Cl, Br, F or I.

2. Process according to Claim 1, characterized in that the coupling reaction is performed at between –20 and +30°C.

3. Process according to Claim 1 or 2, characterized in that the compounds obtained preferably correspond to the following general formula:

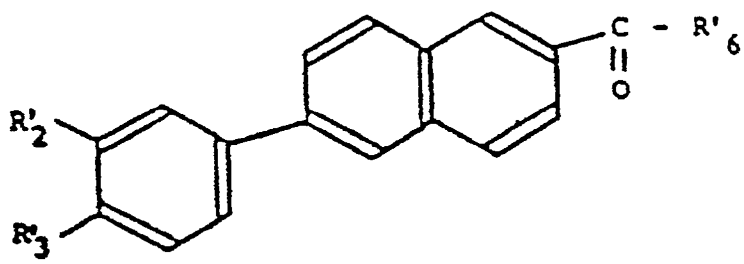

(II)

in which:
$R'_6$ denotes a radical

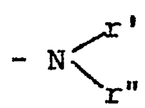

or a radical $-OR'_7$

r' and r", which may be identical or different, denote a hydrogen atom or a lower alkyl radical or, taken together, form a morpholino radical, $R'_7$ denoting a hydrogen atom or a lower alkyl radical,

$R'_2$ denotes a hydrogen atom, an alkyl radical, an alkoxy radical or a 1-adamantyl radical, and

$R'_3$ denotes a hydrogen atom, a hydroxyl radical, an alkyl radical, an alkoxy radical or a 1-adamantytlhio radical.

4. Process according to any one of Claims 1 to 3, characterized in that the compounds obtained are selected from the group consisting of:

- 6-(3-methylphenyl)-2-naphthoic acid and its methyl ester,
- 6-(4-tert-butylphenyl)-2-naphthoic acid and its methyl ester,
- 6-(3-tert-butylphenyl)-2-naphthoic acid and its methyl ester,
- 6-(3,4-dimethoxyphenyl)-2-naphthoic acid and its methyl ester,
- 6-[p-(1-adamantylthio)phenyl]-2-naphthoic acid and its methyl ester,
- 6-[3-(1-adamantyl)-4-methoxyphenyl]-2-naphthoic acid and its methyl ester,
- 6-[3-(1-adamantyl)-4-tert-butyldimethylsilyloxyphenyl]-2-naphthoic acid methyl ester,
- 6-[3-(1-adamantyl)-4-hydroxyphenyl]-2-naphthoic acid methyl ester,
- 6-[3-(1-adamantyl)-4-hydroxyphenyl]-2-naphthoic acid,
- 6-[3-(1-adamantyl)-4-bromo-1-decyloxyphenyl]-2-naphthoic acid methyl ester,
- 6-[3-(1-adamantyl)-4-decyloxyphenyl]-2-naphthoic acid,
- 6-[3-(1-adamantyl)-4-hexyloxyphenyl]-2-naphthoic acid methyl ester,
- 6-[3-(1-adamantyl)-4-hexyloxyphenyl]-2-naphthoic acid,
- 6-[3-(1-adamantyl)-4-methoxyphenyl]-4-acetoxy-1-methyl-2-naphthoic acid methyl ester,
- 6-[3-(1-adamantyl)-4-methoxyphenyl]-4-hydroxy-1-methyl-2-naphthoic acid,
- 6-[3-(1-adamantyl)-4-methoxyphenyl]-4-hydroxy-1-methyl-2-naphthoic acid methyl ester,
- 6-[3-(1-adamantyl)-4-methoxyphenyl]-1-methyl-2-naphthoic acid methyl ester,
- 6-[3-(1-adamantyl)-4-methoxyphenyl]-1-methyl-2-naphthoic acid,
- 6-[3-(1-adamantyl)-4-methoxyphenyl]-2-naphthalenemethanol,

– 6-[3-(1-adamantyl)-4-methoxyphenyl]-2-naphthoic acid ethylamide,
– 6-[3-(1-adamantyl)-4-methoxyphenyl]-2-naphthoic acid morpholide,
– 6-(3-tert-butyl-4-methoxyphenyl)-2-naphthoic acid methyl ester,
– 6-(3-tert-butyl-4-methoxyphenyl)-2-naphthoic acid,
– 6-[3-(1,1-dimethyldecyl)-4-methoxyphenyl]-2-naphthoic acid methyl ester, and
– 6-[3-(1,1-dimethyldecyl)-4-methoxyphenyl]-2-naphthoic acid.

5. Cosmetic composition for body and hair hygiene, characterized in that it contains, in a suitable cosmetic vehicle, at least one active compound of the following general formula:

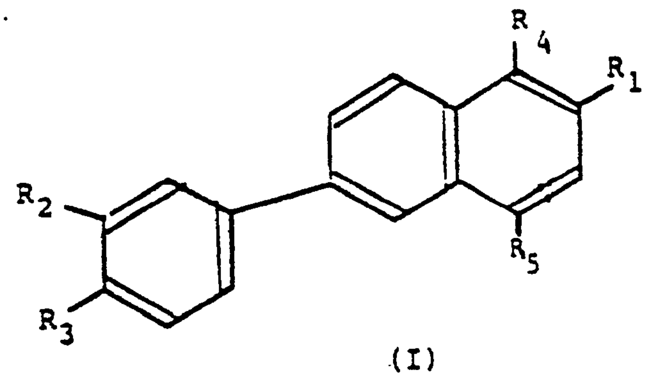

(I)

in which
$R_1$ denotes:

(i) $-\underset{\underset{O}{\|}}{C}-R_6$ ou (ii) $-CH_2OH$

$R_6$ denotes a radical

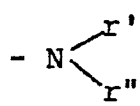

or a radical $-OR_7$, $R_7$ denoting a hydrogen atom, an alkyl radical having from 1 to 20 carbon atoms, a monohydroxyalkyl radical or a polyhydroxyalkyl radical, r' and r" denoting a hydrogen atom, a lower alkyl radical, a mono- or polyhydroxyalkyl radical, an optionally substituted aryl radical or an amino acid residue or amino sugar residue or alternatively taken together form a heterocycle,

$R_2$ denotes a hydrogen atom, an alkyl radical, branched or otherwise, having from 1 to 15 carbon atoms, an alkoxy radical having from 1 to 4 carbon atoms or a cycloaliphatic radical,

$R_3$ denotes a hydrogen atom, a hydroxyl radical, an alkyl radical, branched or otherwise, having from 1 to 4 carbon atoms, an alkoxy radical having from 1 to 10 carbon atoms, a substituted or unsubstituted cycloaliphatic radical, a thiocycloaliphatic radical or a radical of formula $-O-Si(CH_3)_2-R_8$, $R_8$ denoting a linear or branched lower alkyl radical, and

$R_4$ and $Rd_5$, which may be identical or different, denote a hydrogen atom, a lower alkyl radical, a hydroxyl radical or a lower acyloxy radical, and the salts of the said benzonaphthalene derivatives of formula (I).

6. Cosmetic composition according to Claim 5, characterized in that it contains the active compound of formula (I) at a concentration of between 0.0005 and 2%, and preferably between 0.01 and 1%, by weight.